(19) Europäisches Patentamt
European Patent Office
Office européen des brevets

(11) Veröffentlichungsnummer: **0 019 843**
**B1**

# (12) EUROPÄISCHE PATENTSCHRIFT

(45) Veröffentlichungstag der Patentschrift: **29.09.82**

(51) Int. Cl.³: **C 07 H 15/22,** A 61 K 31/70

(21) Anmeldenummer: **80102820.0**

(22) Anmeldetag: **21.05.80**

(54) **Sisomicin-Derivate, Verfahren zu ihrer Herstellung und ihre Verwendung als Arzneimittel.**

(30) Priorität: **30.05.79 DE 2921973**

(43) Veröffentlichungstag der Anmeldung: **10.12.80 Patentblatt 80/25**

(45) Bekanntmachung des Hinweises auf die Patenterteilung: **29.09.82 Patentblatt 82/39**

(84) Benannte Vertragsstaaten: **AT BE CH DE FR GB IT LI NL SE**

(56) Entgegenhaltungen: **EP-A-0 007 996** **FR-A-2 264 553**

**Die Akte enthält technische Angaben, die nach dem Eingang der Anmeldung eingereicht wurden und die nicht in dieser Patentschrift enthalten sind.**

(73) Patentinhaber: **BAYER AG, Zentralbereich Patente, Marken und Lizenzen, D-5090 Leverkusen 1, Bayerwerk (DE)**

(72) Erfinder: **Petersen, Uwe, Dr., Auf dem Forst 4, D-5090 Leverkusen 1 (DE)**
Erfinder: **Metzger, Karl Georg, Dr., Pahlkestrasse 15, D-5600 Wuppertal-1 (DE)**
Erfinder: **Zeiler, Hans-Joachim, Dr., Windrather Strasse 188, D-5620 Velbert 15 (DE)**
Erfinder: **Stadler, Peter, Dr., Ohligser Strasse 85, D-5657 Haan (DE)**
Erfinder: **Voss, Eckart, Dr., Johann Janssen Strasse 38, D-5090 Leverkusen 1 (DE)**

**EP 0 019 843 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Sisomicin-Derivate, Verfahren zu ihrer Herstellung und ihre Verwendung als Arzneimittel

Die vorliegende Erfindung betrifft neue Sisomicin-Derivate, Verfahren zu ihrer Herstellung sowie ihre Verwendung als Arzneimittel.

Sisomicin ist eine antibakteriell wirksame Verbindung der Gruppe der Aminoglykosid-Antibiotika.

Aminoglykosid-Antibiotika sind wichtige Substanzen zur wirkungsvollen Bekämpfung bakterieller Infektionen. Das Auftreten resistenter Keime mindert jedoch in vielen Fällen ihre breite Anwendbarkeit; des weiteren können Nebenwirkungen wie Oto- und Nephrotoxizität auftreten. Durch Derivatisierung gelingt es in einigen Fällen, diese Nachteile zu beseitigen.

So ist es bereits bekannt geworden, dass man durch Acylierung von Sisomicin Verbindungen mit partiell verbesserten Eigenschaften erhalten kann. Beispielsweise ist das 1-N-[(S)-3-Amino-2-hydroxypropionyl]-sisomicin gemäss FR-A 2 264 553 gegenüber einigen Sisomicin-resistenten Bakterien wirksamer als Sisomicin. Gleichzeitig beobachtet man aber infolge dieser chemischen Veränderung am Sisomicinmolekül auch eine reduzierte Wirkung gegenüber sisomicinsensiblen Bakterien (siehe die Veröffentlichung von J.J. Wright et al., J. Antibiot, Tokyo, 29, 714, 1976).

Es wurde nun gefunden, dass die genannten Nachteile in besonders hohem Masse durch die erfindungsgemässen Verbindungen der Formel I

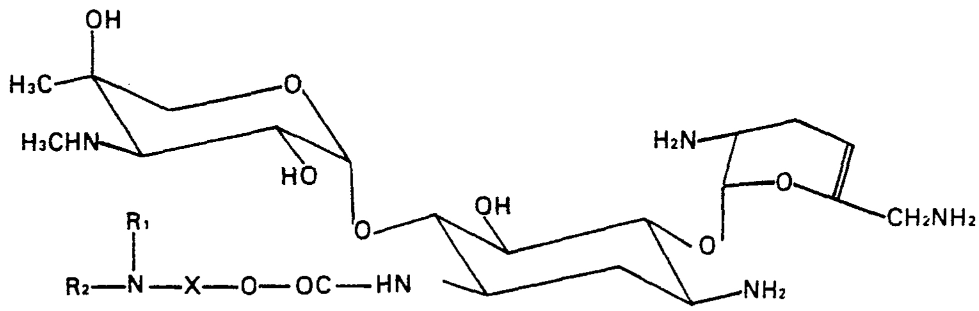

(I)

worin X einen geradkettigen, verzweigten oder cyclischen gesättigten oder ungesättigten aliphatischen Rest mit 2 bis 10 C-Atomen,

$R_1$ Wasserstoff, $C_1$-$C_4$-Alkyl oder Benzyl und

$R_2$ einen Rest der Formel

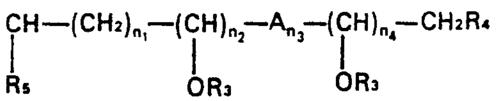

bedeuten, wobei

A für

$$—CH{=}CH—, \quad \left[—CH_{(2-n_5)}—(CH_2OR_3)_{n_5}\right]$$

$R_3$ für Wasserstoff, Triarylmethyl, Alkyl oder Acyl, oder 2 Reste $R_3$ gemeinsam Alkyliden

$R_4$ für Wasserstoff oder $OR_3$,

$R_5$ für Wasserstoff oder gegebenenfalls durch 1-3 Hydroxygruppen substituiertes $C_1$-$C_4$-Alkyl

$n_1$ für 0, 1, 2 oder 3,

$n_2$ für 0, 1, 2, 3, 4 oder 5 und

$n_3$, $n_4$ und $n_5$ unabhängig voneinander für 0, 1 oder 2 stehen, wobei die Summe aus $n_1$, $n_2$, $n_3$ und $n_4$ Werte von 1 bis 5 annimmt und die Gesamtzahl der $OR_3$ Gruppen 1 bis 6 beträgt.

Insbesondere steht X für einen $C_2$ bis $C_6$-Alkylenrest und $R_1$ für Wasserstoff.

Alkyl $R_3$ ist insbesondere $C_1$-$C_4$-Alkyl, Acyl $R_3$ ist insbesondere $C_2$-$C_4$-Alkylcarbonyl, Formyl oder Benzoyl. Alkyliden, gebildet von 2 Resten $R_3$ ist insbesondere $C_1$-$C_6$-Alkyliden. Triarylmethyl $R_3$ ist insbesondere Triphenylmethyl.

Die erfindungsgemässen Verbindungen sowie pharmazeutisch verwendbare Salze zeigen starke antibakterielle Eigenschaften gegen eine Vielzahl von Keimen und eine ausserordentlich gute Verträglichkeit.

Die pharmazeutisch verwendbaren Salze leiten sich insbesondere von anorganischen oder organischen Säuren wie Schwefelsäure, Phosphorsäure, Salpetersäure, Salzsäure, Bromwasserstoffsäure, Essigsäure, Propionsäure, Ascorbinsäure, Zitronensäure usw. ab.

Geeignete Reste $R_2$ sind beispielsweise geradkettige Polyhydroxyalkylreste wie 2,3-Dihydroxypropyl, 2,3,4-Trihydroxybutyl, 2,3,4,5-Tetrahydroxypentyl, 2,3,4,5,6-Pentahydroxyhexyl, 3,4-Dihydroxybutyl, 3,4,5-Trihydroxypentyl, 3,4,5,6-Tetrahydroxyhexyl, 4,5-Dihydroxypentyl, 4,5,6-Trihydroxyhexyl, 4,5-Dihydroxyhexyl, 2,3,4-Trihydroxypentyl, 2,3,4,5-Tetrahydroxyhexyl, 3,4,5,6,7-Pentahydroxyheptyl, 3,4,5,6-Tetrahydroxyheptyl, 2,4,5-Trihydroxypentyl, 2,4,5,6-Tetrahydroxyhexyl, 2,4,5-Trihydroxyhexyl, 2,5-Dihydroxypentyl, 2,3-Dihydroxypentyl, verzweigtkettige Polyhydroxyalkylreste wie 2,4-Dihydroxy-3-hydroxymethylpentyl, 2,2-Bishydroxymethylpropyl, geradkettige Polyhydroxyalkenylreste wie 4,5-Dihydroxy-pent-2-en-1-yl, 4,5,6-Trihydroxy-hex-2-en-1-yl, 4,5-Dihydroxy-hex-2-en-1-yl sowie an den OH-Gruppen acylierte und alkylierte Polyhydroxyalkylgruppen wie 2,3,4,5-Tetraacetyloxypentyl, 2,3,4,5-Tetrabenzoyloxyhexyl, 2,3-Dimethoxypropyl, 2,3,4-Tri-

hydroxy-5-methoxypentyl, 2,3-O-Isopropylidenpropyl, 2,3-O-Cyclohexylidenpropyl, 1-Hydroxymethyl-ethyl, 2-Hydroxy-1-hydroxymethyl-ethyl, 3-Hydroxy-1-methyl-propyl, 3-Hydroxy-1-hydroxymethyl-propyl, 2,3-Dihydroxy-1-hydroxymethyl-propyl, 4-Hydroxy-1-methyl-butyl, 4-Hydroxy-1-hydroxymethyl-butyl, 2,4-Dihydroxy-1-hydroxymethyl-butyl, 2,3,4-Trihydroxy-1-hydroxymethyl-butyl, 1-Hydroxymethyl-propyl, 3-Hydroxy-1-(2-hydroxyethyl)-propyl, 1-Hydroxymethyl-butyl, 3,4-Dihydroxy-1-(1,2-dihydroxyethyl)-butyl.

Die vorstehend aufgeführten Reste sind lediglich beispielhaft zu verstehen. Sie enthalten alle mindestens ein – in den meisten Fällen mehrere – chirale C-Atome und liegen als optisch reine Diastereomere oder Diastereomerengemische vor. Es kann von Vorteil sein, die erfindungsgemässen Verbindungen als optisch reine Produkte zu verwenden.

Ganz besonders bevorzugt sind Verbindungen der Formel I, in denen $R_3$ Wasserstoff bedeutet.

Es wurde weiterhin gefunden, dass man die neuen Verbindungen der Formel I, in denen $R_1$ Wasserstoff bedeutet, erhält, wenn man eine Verbindung der Formel II

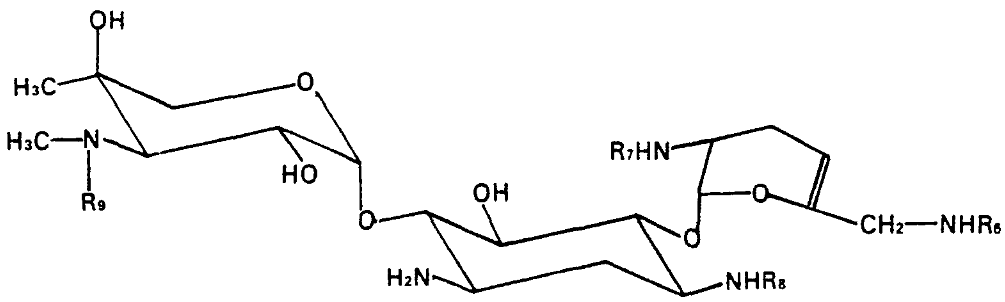

(II)

worin

$R_6$, $R_7$ und $R_8$ für $-CO-R_{10}$ oder $-S-R_{11}$ und

$R_9$ für $-S-R_{11}$ stehen,

wobei $R_{10}$ einen Rest der Formeln

$-CHal_3$, $-(CH_2)_{n_6}B$, $-O-E$,

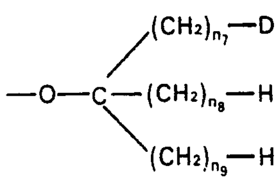

oder

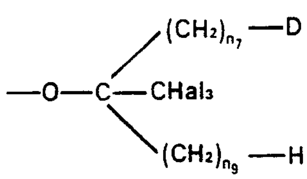

B und D Wasserstoff oder gegebenenfalls substituiertes Phenyl,

E gegebenenfalls substituiertes Phenyl,

$n_6$ eine Zahl von 0–5,

$n_7$, $n_8$ und $n_9$ eine Zahl von 0–5,

Hal Fluor, Chlor oder Brom und

$R_{11}$ gegebenenfalls substituiertes Phenyl, Di- oder Triphenylmethyl

bedeuten,

in an sich bekannter Weise mit einem Acylierungsmittel der Formel III

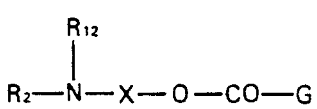

(III)

worin

$R_2$ und X die oben angegebene Bedeutung haben und

G für eine Abgangsgruppe, vorzugsweise für Halogen wie Chlor und Brom, Azido oder gegebenenfalls substituiertes Phenoxy oder einen Rest der Formel

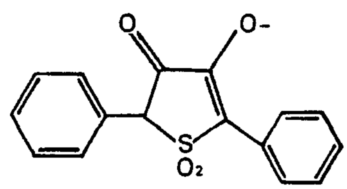

und $R_{12}$ für $-CO-R_{10}$ oder $-S-R_{11}$ stehen,

umsetzt, die Schutzgruppen $R_6$, $R_7$, $R_8$, $R_9$ und $R_{12}$ sowie gegebenenfalls Alkylidengruppen, die 2 Reste $R_3$ gemeinsam bilden, in üblicher Weise abspaltet und die resultierenden Verbindungen gegebenenfalls in ihre pharmazeutisch verwendbaren Salze überführt.

Gegebenenfalls substituiertes Phenoxy ist insbesondere 4-Nitrophenoxy, Phenoxy und 2,4,5-Trichlorphenoxy.

Geeignete Substituenten der gegebenenfalls substituierten Phenyl-, Di- oder Triphenylmethylreste $R_{11}$ sind z.B. 1 bis 3 Substituenten aus der Reihe Trifluormethyl, Nitro, $C_1$–$C_4$-Alkyl, $C_1$–$C_4$-Alkoxy, $C_1$–$C_4$-Alkoxycarbonyl oder Phenyl oder 1 bis 5 Halogenatome, vorzugsweise Chloratome. Als Beispiele für $-SR_{11}$-Gruppen seien o-Nitrophenylsulphenyl und 2,4,5-Trichlorphenylsulphenyl genannt.

Geeignete Substituenten der gegebenenfalls substituierten Phenylreste B, D und E sind 1 oder 2 Substituenten aus der Reihe Nitro, Halogen, vorzugsweise Chlor, $C_1$–$C_4$-Alkyl, $C_1$–$C_4$-Alkoxy und Phenyl.

Geeignete Acylierungsmittel (III) sind z.B.

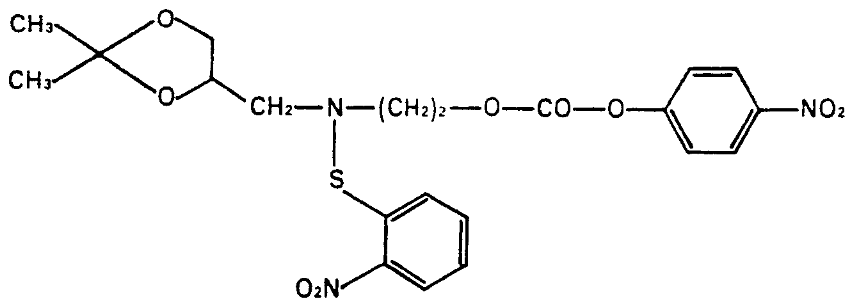

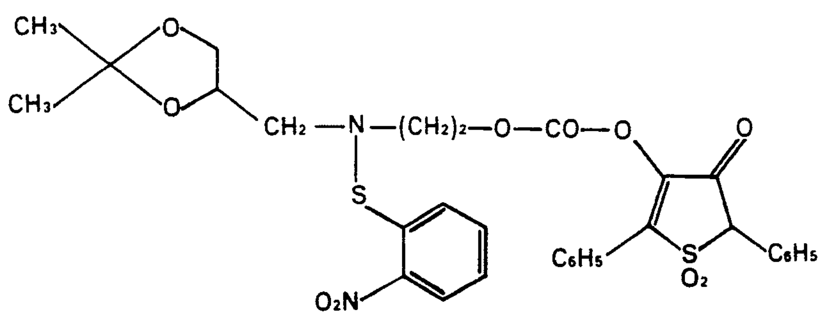

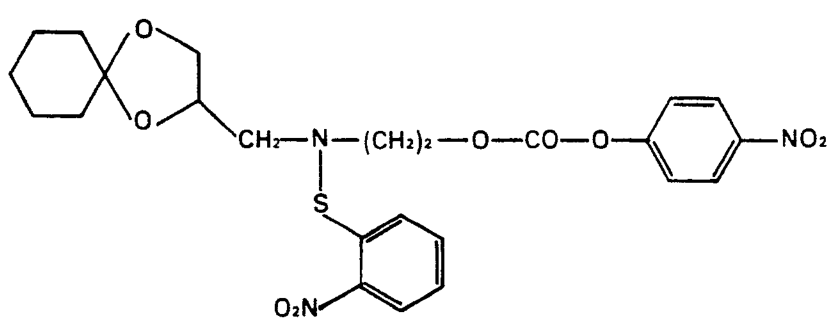

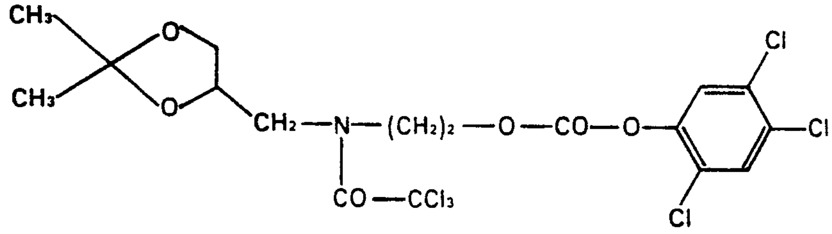

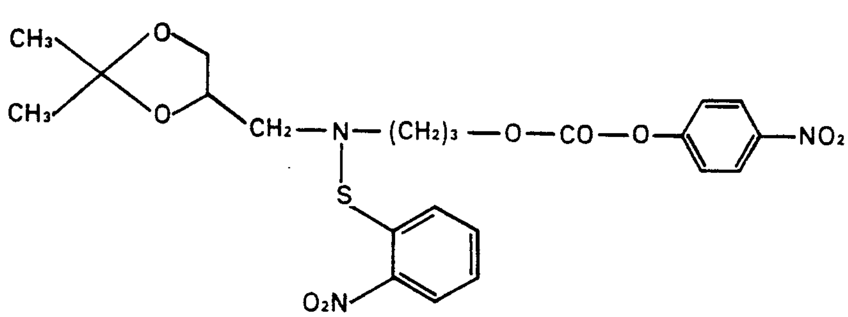

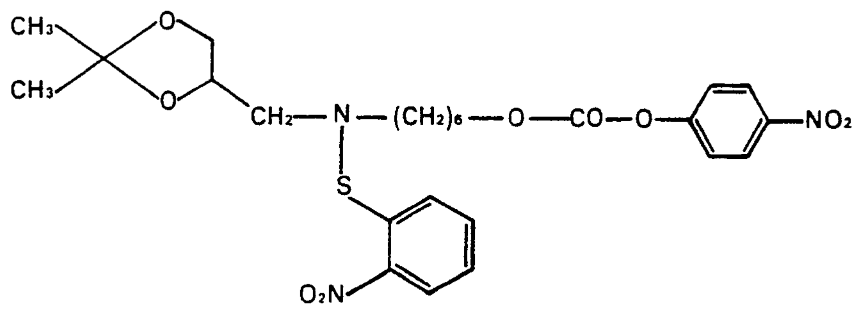

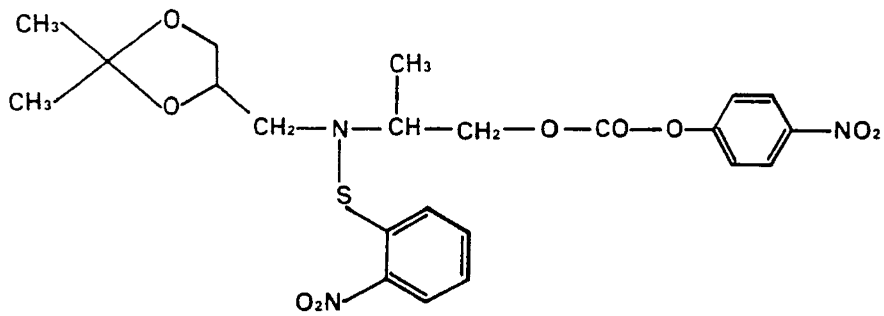

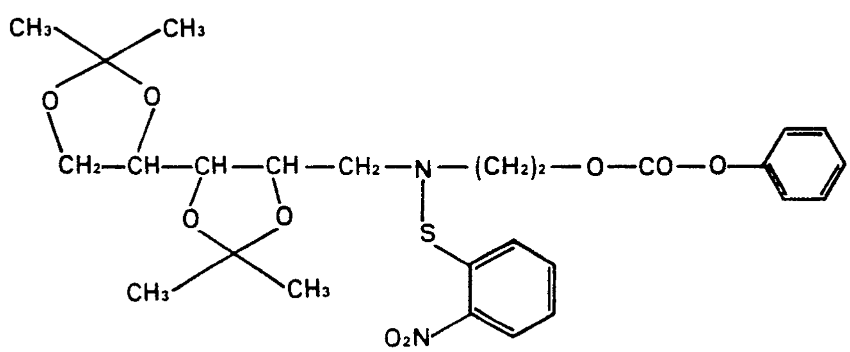

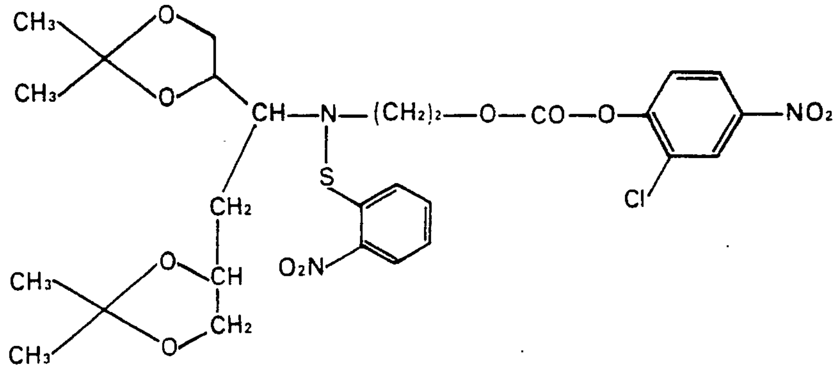

Darüber hinaus erhält man die Verbindungen der Formel I, wenn man eine Verbindung der Formel IV

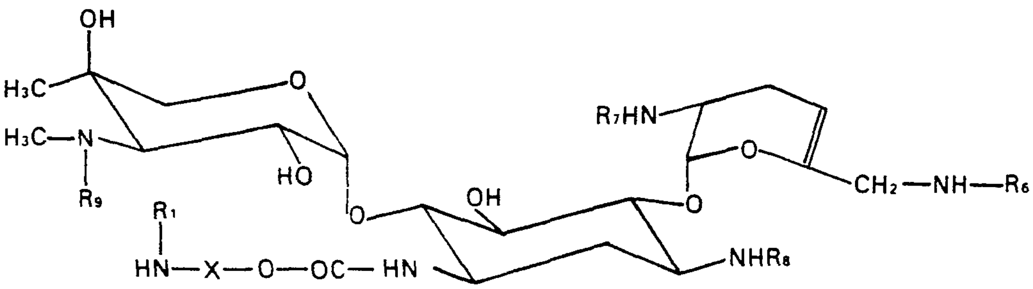

(IV)

worin
$R_1$, $R_6$, $R_7$, $R_8$, $R_9$ und X die oben angegebene Bedeutung haben
mit Carbonylverbindungen der Formel V

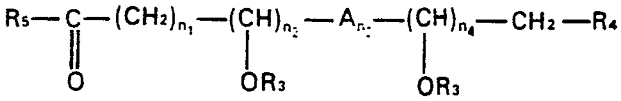

(V)

worin
$R_3$, $R_4$, $R_5$, A, $n_1$, $n_2$, $n_3$ und $n_4$ die bereits genannte Bedeutung haben,
in Gegenwart eines Wasserstoffdonorreduktionsmittels umsetzt und anschliessend die Schutzgruppen $R_6$, $R_7$, $R_8$ und $R_9$ sowie gegebenenfalls Alkylidengruppen, die 2 Reste $R_3$ gemeinsam bilden, abspaltet und die erhaltenen Produkte gegebenenfalls in ihre pharmazeutisch verwendbaren Salze überführt.

Bei den erfindungsgemäss verwendeten Carbonylverbindungen der Formel V handelt es sich z.B. um Glykolaldehyd, um Aldehydozucker wie D- oder L-Glycerinaldehyd, D-Erythorse, D-Ribose, D- oder L-Arabinose, D-Glucose, D-Galaktose, 2-Desoxy-D-ribose, 2-Desoxy-D-glucose, 2-Desoxy-D-galaktose, 6-Desoxy-L-mannose, 6-Desoxy-D-glucose, 5-Desoxy-D-ribose, 2,6-Di-desoxy-D-glucose bzw. -L-glucose, 3-O-Methyl-D-glucose, 5-O-Methyl-D-ribose und um Ketone wie z.B. Hydroxyaceton, Methoxyaceton, Dihydroxyaceton, 4-Hydroxy-2-butanon, 1,4-Dihydroxy-2-butanon, 1,3,4-Trihydroxy-2-butanon, 5-Hydroxy-2-pentanon, 1,5-Dihydroxy-2-pentanon, 1,3,5-Trihydroxy-2-pentanon, 1,3,4,5-Tetrahydroxy-2-pentanon, 1-Hydroxy-2-butanon, 1,5-Dihydroxy-3-pentanon, 1-Hydroxy-2-pentanon, 1,2,5,6-Tetrahydroxy-3-hexanon. Darüber hinaus können auch Carbonylverbindungen verwendet werden, deren Hydroxylgruppen durch geeignete Schutzgruppen blockiert sind. Beispielhaft seien genannt: 2,3-O-Isopropyliden-D-glycerinaldehyd, 2,3-O-Cyclohexyliden-D-glycerinaldehyd, 4,5-O-Cyclohexyliden-2,3-didesoxy-D,L-ribose, 4-(Tetrahydropyran-2-yloxy)-2-buten-1-al.

Als Ausgangsstoffe der Formel (II) dienen bevorzugt 2',3,3'',6'-Tetra-N-(o-nitrophenylsulfenyl)-sisomicin, 3''-N-(o-Nitrophenylsulfenyl)-2',3,6'-tris-N-trichloracetyl-sisomicin, 3''-N-(o-Nitrophenylsulfenyl)-2',3,6'-tris-N-trifluoracetyl-sisomicin, 3''-N-(o-Nitrophenylsulfenyl)-2',3,6'-tris-N-(2,2,2-trichlorethoxycarbonyl)-sisomicin, 3''-N-(o-Nitrophenylsulfenyl)-2',3,6'-tris-N-(1,1-dimethyl-2,2,2-trichlorethoxycarbonyl)-sisomicin, 3''-N-(o-Nitrophenylsulfenyl)-2',3,6'-tris-N-(4-methoxybenzyloxycarbonyl)-sisomicin, 3''-N-(o-Nitrophenylsulfenyl)-2',3,6'-tris-N-phenoxycarbonylsisomicin, 3''-N-(o-Nitrophenylsulfenyl)-2',3,6'-tris-N-(tert.-butoxycarbonyl)-sisomicin, deren Herstellung nach dem in DE-OS 27 26 197 beschriebenen Verfahren beziehungsweise über die folgenden Stufen erfolgt:

1. Umsetzung von Penta-N-(o-nitrophenylsulfenyl)-sisomicin (Deutsche Offenlegungsschrift 27 26 197) mit Dimethyl-(1,2-dimethylpropyl)-silylchlorid zum Penta-N-(o-nitrophenylsulfenyl)-2''-O-[dimethyl-(1,2-dimethylpropyl)-silyl]-sisomicin;
2. Abspaltung der 0-Nitrophenylsulfenylgruppen von 2',3,6'-N mit 2-Mercaptobenthiazol;
3. Acylierung der 2',3,6'-N-Positionen mit einem üblichen Acylierungsmittel;
4. Abspaltung der 2''-O-Schutzgruppe und
5. Abspaltung der 1-N-(o-Nitrophenylsulfenyl)-Gruppe.

Die als Zwischenprodukt verwendeten Verbindungen der Formel IV werden erhalten, wenn man eine Verbindung der Formel II mit einem Acylierungsmittel der Formel VI

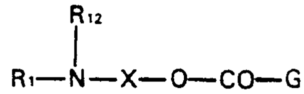

(VI),

in welcher
$R_1$, $R_{12}$, X und G die oben angegebene Bedeutung haben, zu einer Verbindung der Formel VII

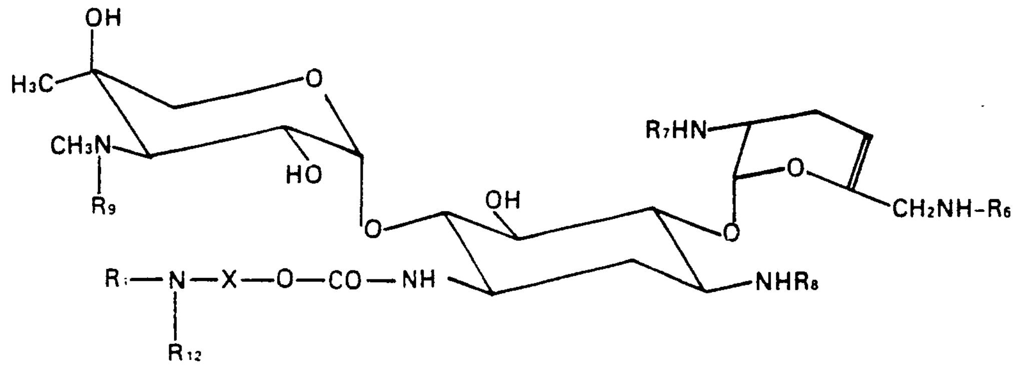

(VII)

worin

$R_1$, $R_6$, $R_7$, $R_8$, $R_9$, $R_{12}$ und X die oben genannten Bedeutungen haben, umsetzt und anschliessend die Schutzgruppe $R_{12}$ selektiv in Gegenwart der Schutzgruppen $R_6$, $R_7$, $R_8$ und $R_9$ abspaltet.

Die als Acylierungsmittel verwendeten Verbindungen der Formel (VI) lassen sich nach prinzipiell bekannten Verfahren durch Verknüpfung eines Aminoalkohols mit der Schutzgruppe $R_{12}$ an der Aminogruppe und Überführung der alkoholischen Funktion in ein reaktives Carbonat herstellen. Als Beispiele seien genannt:

(4-Nitrophenyl)-(2-trifluoracetylaminoethyl)-carbonat,
(2,4,5-Trichlorphenyl)-(3-trifluoracetylaminopropyl-1)-carbonat,
(4-Nitrophenyl)-[2-(2-nitrophenylsulfenylamino)-ethyl]-carbonat,
(4-Nitrophenyl)-(6-trifluoracetylaminohexyl-1)-carbonat,
(4-Nitrophenyl)-(2-trichloracetylaminoethyl)-carbonat,
(2,4,5-Trichlorphenyl)-(4-trichloracetylbutyl-1)-carbonat,
(4-Nitrophenyl)-(2-trichloracetylaminopropyl-1)-carbonat,
(4-Nitrophenyl)-(2-trifluoracetyl-methylaminoethyl)-carbonat,
(4-Nitrophenyl)-(2-trichloracetyl-benzylaminoethyl)-carbonat,
Chlorameisensäure-(2-trichloracetylaminoethyl)-ester.

Bei der Durchführung des erfindungsgemässen Verfahrens setzt man nach der erstgenannten Methode 1 Mol der Verbindung der Formel II mit etwa 1 bis 3 Mol, vorzugsweise 1,1 bis 1,5 Mol einer Verbindung der Formel III um, wobei alle inerten organischen Lösungsmittel wie Toluol, Chloroform, Methylenchlorid, Dimethylformamid, Dimethylacetamid, Dimethylsulfoxid, Äther wie Diäthyläther, Dioxan und Tetrahydrofuran, Pyridin, Alkohole wie Methanol und Äthanol sowie deren Gemischen als Verdünnungsmittel in Frage kommen.

Werden Säurebinder benötigt, so können alle üblichen organischen und anorganischen Säurebindungsmittel verwendet werden. Hierzu gehören vorzugsweise Alkali- und Erdalkalihydroxide wie Natriumhydroxid, Kaliumhydroxid oder Calciumhydroxid, Alkali- und Erdalkalicarbonate und -hydrogencarbonate wie Natriumcarbonat, Kaliumcarbonat, Nariumhydrogencarbonat und Calciumcarbonat, Calciumoxid, tertiäre aliphatische und aromatische Amine wie Triäthylamin und N,N-Dimethylanilin sowie heterocyclische Basen wie Pyridin und Chinolin.

Die Reaktionstemperaturen können in einem grossen Bereich variiert werden. Im allgemeinen arbeitet man bei Temperaturen von etwa -30°C bis +80°C, vorzugsweise zwischen etwa 0°C und etwa +40°C.

Die Umsetzung kann bei Normaldruck, aber auch bei erhöhtem Druck durchgeführt werden. Im allgemeinen arbeitet man bei Normaldruck.

Für die Durchführung des erfindungsgemässen Verfahren nach der zweiten Methode setzt man 1 Mol einer Verbindung der Formel IV mit etwa 1 bis 3 Mol, vorzugsweise 1,1 bis 1,5 Mol einer Verbindung der Formel V in Gegenwart von 1 bis 3 Mol, vorzugsweise 1,1 bis 1,5 Mol eines Wasserstoff-Donor-Reduktionsmittels um.

Wasserstoff-Donor-Reduktionsmittel, die bei diesem Verfahren Verwendung finden, umfassen Dialkylaminborane, z.B. Dimethylaminboran, Diethylaminboran und Morpholinboran, Tetraalkylammoniumcyanoborhydride, z.B. Tetrabutylammoniumcyanoborhydrid, Alkalimetallborhydride, z.B. Natriumborhydrid und vorzugsweise Alkalimetallcyanoborhydride, Lithiumcyanoborhydrid und Natriumcyanoborhydrid.

Das Verfahren wird üblicherweise in einem inerten Lösungsmittel durchgeführt. Das Lösungsmittel kann ein organisches oder anorganisches sein, in dem die selektiv geschützte Verbindung der Formel IV und die anderen Reagentien löslich sind und das unter den Reaktionsbedingungen nach Möglichkeit Nebenreaktionen herabsetzt oder verhindert. Obwohl wasserfreie aprotische Lösungsmittel mit Vorteil eingesetzt werden können, z.B. Tetrahydrofuran, wenn das Reduktionsmittel Morpholinboran ist, wird gewöhnlich doch ein protisches Lösungsmittel verwendet. Als solches eignet sich z.B. ein niederes Alkanol oder vorzugsweise Wasser oder ein wässriges niedriges Alkanol, vorzugsweise wässriges Methanol, Ethanol oder andere Lösungsmittelsysteme, die Wasser enthalten wie wässriges Dimethylformamid, wässriges Hexamethylphosphoramid, wässriges Tetrahydrofuran oder wässriger Ethylenglycoldimethylether.

Das Verfahren wird gewöhnlich in einem

pH-Bereich von 1 bis 11 und vorzugsweise bei pH 4 bis 8 durchgeführt.

Die reduktive Alkylierung mit einer Carbonylverbindung der Formel V in Gegenwart eines Wasserstoff-Donor-Reduktionsmittels wird gewöhnlich bei Raumtemperatur in Gegenwart von Luft durchgeführt, obwohl es günstiger sein kann, die Reaktion unter Inertgas (Argon, Stickstoff) durchzuführen. Die Reaktion ist gewöhnlich sehr rasch, oft in weniger als 60 Minuten, vollendet, was durch dünnschichtchromatographische Bestimmungen festgestellt werden kann.

Nach beendeter Umsetzung der Verbindungen II mit III beziehungsweise IV mit V werden die im Molekül enthaltenen Schutzgruppen entfernt.

Die Abspaltung der Sulfenylschutzgruppen kann mit schwachen Säuren oder mit schwefelhaltigen, nukleophilen Reagentien wie beispielsweise $H_2S$, Thiophenol oder 2-Mercaptobenzthiazol erfolgen, die Abspaltung der übrigen Schutzgruppen kann mit wässrigem Alkali- oder Erdalkalihydroxid oder mit Säuren wie Trifluoressigsäure, Perchlorsäure oder Bortrifluoridätherat erfolgen.

Verwendet man 3″-N-(o-nitrophenylsulfenyl)-3,2′,6′-tris-N-(trichloracetyl)-sisomicin und |2-[N-(2,3-O-Cyclohexylidendioxypropyl)-N-(o-nitrophenylsulfenyl)-amino]-ethyl|-(4-nitrophenyl)-carbonat als Ausgangsstoffe, so kann der Reaktionsablauf durch das folgende Formelschema als Beispiel für die erstgenannte Methode wiedergegeben werden.

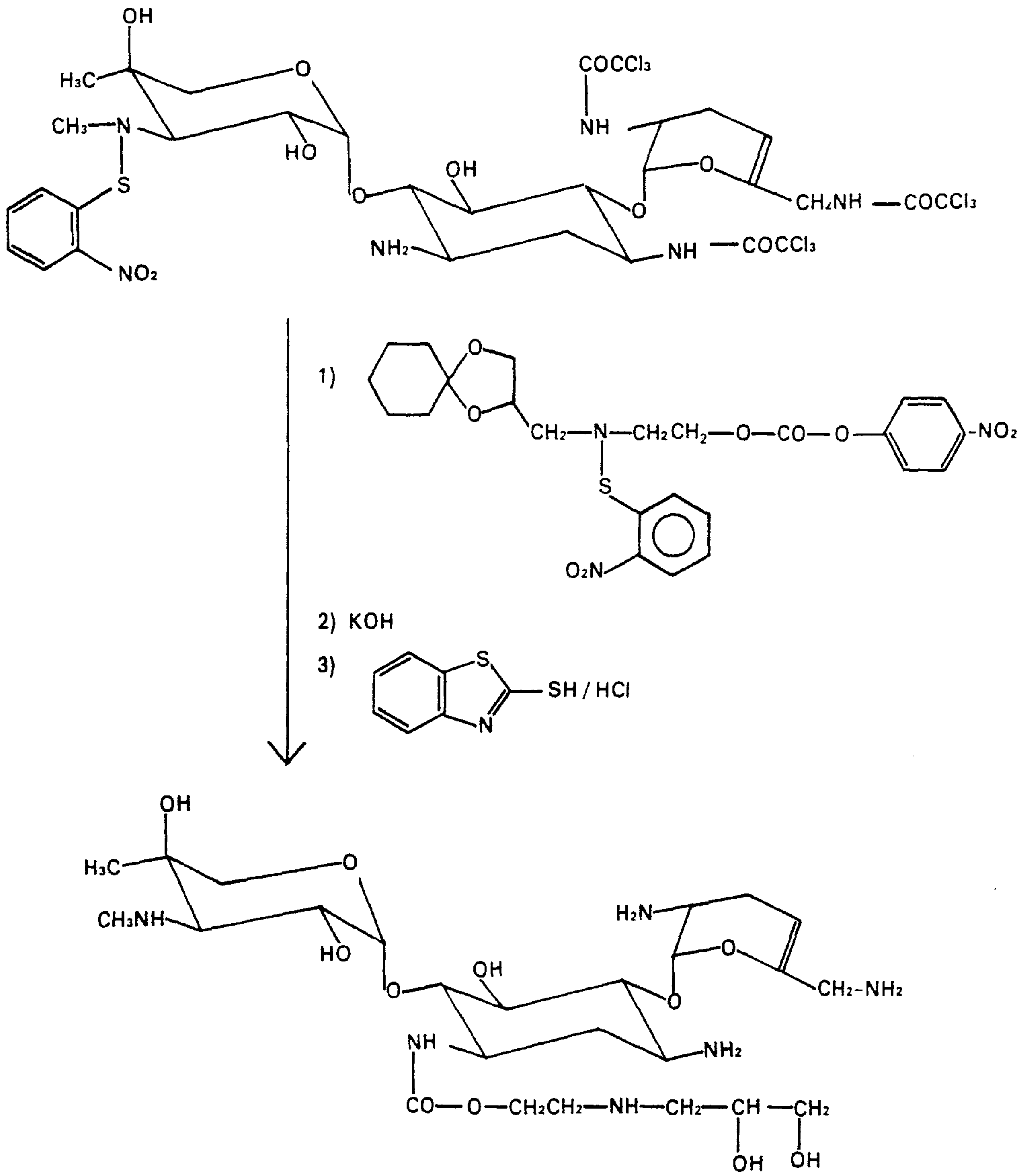

Verwendet man 1-N-(2-Aminoethyloxycarbonyl)-3,2',6',3"-tetra-N-(o-nitrophenylsulfenyl)-sisomicin und Dihydroxyaceton als Ausgangsstoffe, so kann der Reaktionsablauf als Beispiel für die zweite Methode durch das folgende Formelschema wiedergegeben werden:

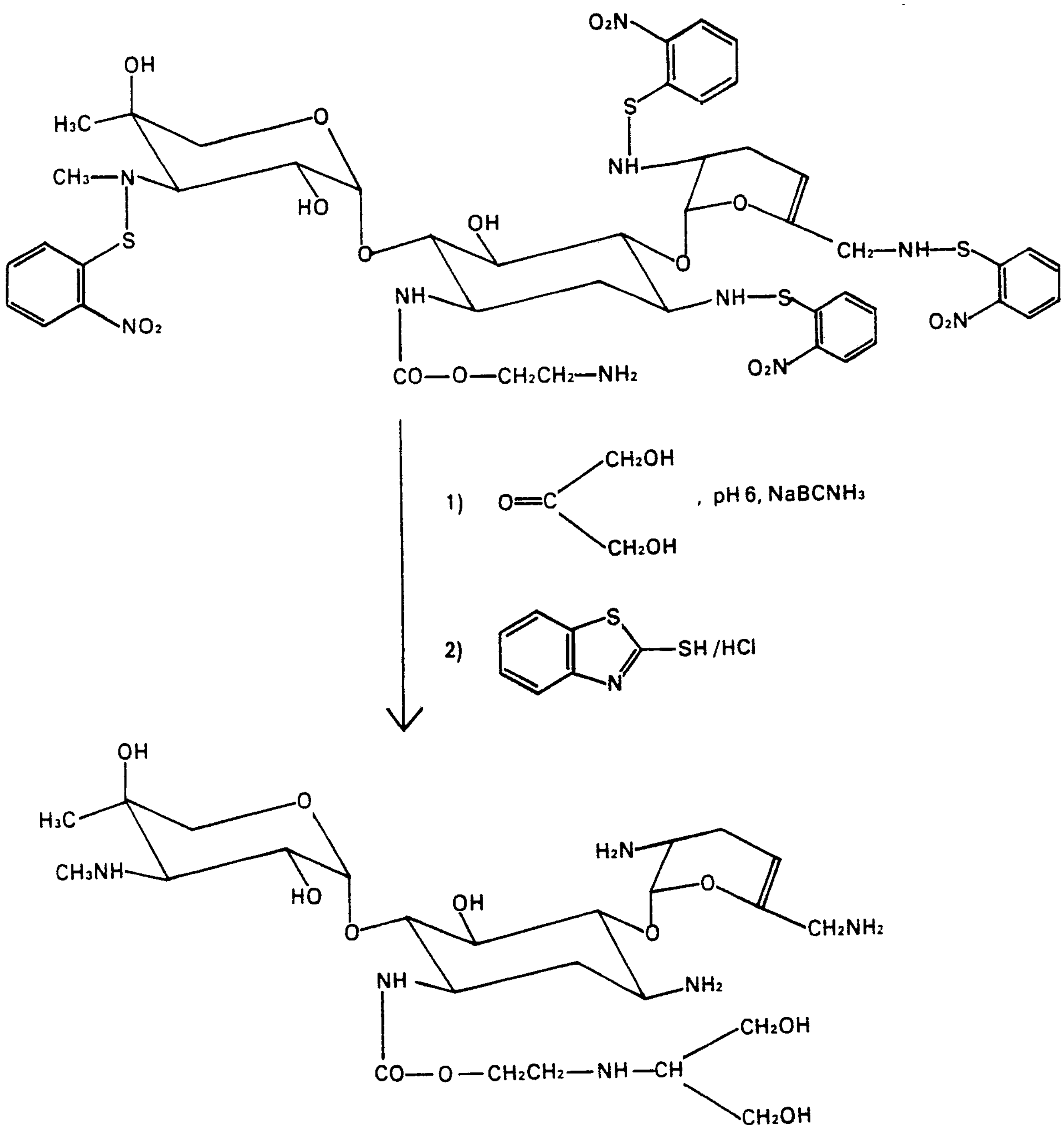


Die erfindungsgemässen Verbindungen sind antimikrobielle Mittel mit einem breiten Wirkungsspektrum und besonderer Wirksamkeit gegen gram-negative Bakterien. Diese Eigenschaften ermöglichen ihre Verwendung als Arzneimittel, insbesondere bei der Bekämpfung von durch Bakterien hervorgerufenen Erkrankungen bei Mensch und Tier. Sie sind gut zur Prophylaxe und Chemotherapie von lokalen und systemischen Infektionen insbesondere Infektionen des Urogenitalsystems in der Human- und Tiermedizin geeignet, die durch gram-negative Bakterien, z.B. E. coli, Proteus, Klebsiella und Pseudomonas verursacht werden, geeignet. Hemmhöfe im Agar-Lochtest wurden z.B. gegen folgende Bakterienstämme bei einer Konzentration von 100 Mikrogramm/1 ml gefunden:

| | |
|---|---|
| Pseudomonas aerug. | 5737 |
| Pseudomonas aerug. | F 41 |
| Klebsiella pneum. | 2 München |
| Klebsiella pneum. | 1 Düsseldorf |
| E. coli | Münster |
| E. coli | Neumann |

Das Verfahren zur Behandlung von durch Bakterien hervorgerufenen Erkrankungen ist dadurch gekennzeichnet, dass man die neuen 1-N-Derivate Menschen und Tieren appliziert, die an diesen Erkrankungen leiden.

Im allgemeinen hängt die zu verabreichende Dosis der Verbindungen der Erfindung vom Alter

und Gewicht des Lebewesens, von der Verabreichungsart, vom Typ und von der Schwere der bakteriellen Infektion ab. Die Dosierung der erfindungsgemässen Verbindungen ist gewöhnlich der Dosierung der 1-N-unsubsitituierten Verbindungen ähnlich. Der Dosierungsspielraum beträgt von 20 mg/Tag/Mensch bis 2000 mg/Tag/Mensch, vorzugsweise 100 mg-500 mg/Tag.

Die Verbindungen der Erfindung können oral verabreicht werden. Die Verabreichung kann in Einzelgaben oder auf mehrere Gaben verteilt erfolgen. Sie können auch topisch verabreicht werden in der Form von Salben, Cremen oder Lotionen. Pharmazeutische Trägerstoffe für diese Formulierungen umfassen Wasser, Öle, Fette, Polyester und Polyole.

Wie im folgenden gezeigt wird, ist das Verfahren zur Herstellung von Arzneimitteln, welche die neuen 1-N-Sisomicin-Derivate enthalten, dadurch gekennzeichnet, dass man die neuen Verbindungen mit pharmazeutisch geeigneten Träger und/oder Zusatzstoffen vermischt.

Zur oralen Verabreichung der Verbindungen dieser Erfindung können Tabletten, Kapseln oder Elixiere Anwendung finden, die Verbindungen können aber auch dem Tierfutter zugemischt werden.

Im allgemeinen enthalten topische Präparationen ca. 0,1 bis ca. 3,0 g der Verbindungen der Erfindung pro 100 g Salbe, Creme oder Lotion. Die topische Verabreichung erfolgt ca. 2 bis 5mal am Tag.

Die antibakteriellen Mittel der Erfindung können in flüssiger Form als Lösungen oder Suspensionen zur Anwendung an Ohren und Augen oder zur parenteralen Verabreichung in Form intramuskulärer oder intravenöser Injektionen vorliegen. Injektionslösungen oder -suspensionen werden gewöhnlich so verabreicht, dass ca. 1 bis 15 mg Wirkstoff pro Kilogramm Körpergewicht in 2 bis 4 Dosen pro Tag in den infizierten Organismus gelangen. Die genaue Dosis hängt von der Art der Infektionen, der Empfindlichkeit des infizierenden Keims und von den individuellen Charakteristika des zu Behandelnden ab.

Formulierung 1

| Tablette | 10 mg Tab. | 25 mg Tab. | 100 mg Tab. |
|---|---|---|---|
| a) Verbindung nach Beispiel 31 | 10,50 mg* | 26,25 mg* | 105,00 mg* |
| Lactose | 197,50 mg | 171,25 mg | 126,00 mg |
| Maisstärke | 25,00 mg | 25,00 mg | 35,00 mg |
| Polyvinylpyrrolidon | 7,50 mg | 7,50 mg | 7,50 mg |
| Magnesiumstearat | 2,50 mg | 2,50 mg | 3,50 mg |

* 5%iger Überschuss

Zur Herstellung der Tabletten wird eine Aufschlämmung der Verbindung nach Beispiel 31, Lactose und Polyvinylpyrrolidon, hergestellt und diese sprühgetrocknet. Man gibt die Maisstärke und Magnesiumstearat zu und verpresst zu Tabletten.

Formulierung 2

| Salbe | |
|---|---|
| Verbindung nach Beispiel 31 | 1,0 g |
| Methylparaben U.S.P. | 0,5 g |
| Propylparaben U.S.P. | 0,1 g |
| Petrolatum | auf 1000 g |

Herstellung

1. Man schmilzt das Petrolatum;
2. Man mischt die Verbindung nach Beispiel 31 Methylparaben und Propylparaben mit ca. 10% des geschmolzenen Petrolatum;
3. Gibt das Gemisch in eine Kolloidmühle;
4. Gibt das restliche Petrolatum unter Rühren zu und kühlt bis es halbfest wird. Das Produkt wird in geeignete Behälter abgefüllt.

Formulierung 3

| Injektionslösung | Per 2,0 ml Viole | per 50 Liter |
|---|---|---|
| Verbindung nach Beisp. 31 | 84,0 mg* | 2100,0 gm |
| Mathylparaben, U.S.P. | 3,6 mg | 90,0 gm |
| Propylparaben, U.S.P. | 0,4 mg | 10,0 gm |
| Natriumbisulfit, U.S.P. | 6,4 mg | 160,0 gm |
| Dinatriumäthylendiamintetraacetat-dihydrat | 0,2 mg | 5,0 gm |
| Wasser, U.S.P. q.s. | 2,0 mg | 50,0 Liter |

* 5%iger Überschuss

Beispiel 1

Penta-N-(o-nitrophenylsulfenyl)-2″-O-[dimethyl-(1,2-dimethyl-propyl)-silyl]-sisomicin

60,6 g rohes Penta-N-(o-nitrophenylsulfenyl)-sisomicin (DE-OS 27 26 197) und 8,75 g Imidazol werden in 250 ml absolutem Dichlormethan gelöst. Bei 0°C werden unter Feuchtigkeitsausschluss 22,5 ml Dimethyl-(1,2-dimethylpropyl)-silyl-chlorid zugetropft. Der Ansatz wird im Vakuum auf ca. 170 ml eingedampft und bei Raumtemperatur 48 Stunden stehen gelassen. Nach Zugabe von 130 ml absolutem Dichlormethan wird der Niederschlag abgesaugt, das Filtrat mit 350 ml Petroläther kräftig durchgeschüttelt und die Petrolätherphase abdekantiert und verworfen. Das ausgefallene Öl wird in 100 ml Dichlormethan gelöst, mit 250 ml Petroläther erneut ausgefällt und schliesslich im Hochvakuum getrocknet. Ausbeute 60 g (89%) Rohprodukt, das ohne weitere Reinigung für die weiteren Umsetzungen eingesetzt wird. Ein reines Präparat wird durch Chromatographie an Kieselgel mit $CH_2Cl_2/CH_3OH$ = 99/1 erhalten.

13-C-NMR ($CDCl_3$)
δ = 124–138 (arom. C)
147,54 (C-5′); 102,26 (C-1″);
97,81 (C-4′); 99,09 (C-1′);
-2,9 bis 3,0 ($Si-CH_3$);
22,77 (Si-CH-); 30,60 (Si-CH-CH) ppm

Als Nebenprodukt wird das Penta-N-(o-nitrophenylsulfenyl)-2″, 5- bis -O-[dimethyl-(1,2-dimethyl-propyl-silyl]-sisomicin isoliert.

13-C-NMR ($CDCl_3$)
δ = 124–146 (arom. C),
148,00 (C-5′); 96,13 (C-4′) ppm

Beispiel 2

1,3″-Bis-N-(o-Nitrophenylsulfenyl)-2″-O-[dimethyl-(1,2-dimethyl-propyl)-silyl]-sisomicin

56 g rohes Penta-N-(o-nitrophenylsulfenyl)-2″-O-[dimethyl-(1,2-dimethyl-propyl-silyl]-sisomicin in 36 ml Dichlormethan/70 ml Methanol werden mit 16 g 2-Mercapto-benzthiazol versetz, geschüttelt, bis klare Lösung erfolgt und bei 5°C 2 Stunden stehen gelassen. Der sich dabei abscheidende Niederschlag wird abfiltriert und die Lösung ohne Isolierung des gewünschten Produktes für die weiteren Umsetzungen verwendet. Die Ausbeute beträgt etwa 80% der Theorie. Zur Darstellung eines reinen Präparats wird das Filtrat rasch im Vakuum eingedampft und der Rückstand mit a) Dichlormethan, b) Dichlormethan/$CH_3OH$ (8:2) und c) mit $CH_2Cl_2/CH_3OH$/20% wässrigem Ammoniak (7:2, 7:0,3), an Kieselgel chromatographiert. Die Ausbeute an reinem Produkt beträgt 25,3 g (69%).

13-C-NMR ($CD_3OD$)
δ = 1,5 ($Si-CH_3$); 122–146 (arom. C),
147,14 (C-5′); 103,31 (C-1″); 100,16 (C-1′)
99,30 (C-4′) ppm

Als Nebenprodukt werden bei der Säulenchromatographie 3 g (10%) 3″-N-(o-Nitrophenylsulfenyl)-2″-O-[dimethyl-(1,2-dimethyl-propyl)-silyl]-sisomicin isoliert.

13-C-NMR ($CD_3OD$):
δ = 76,66 (C-2″); 21,70 (C-6″); 30,40 ($N-CH_3$);
53,13 (C-1), 52,18 (C-3); 44,06 (C-6′)
49,41 (C-2′) ppm

Beispiel 3

1,3″-Bis-N-(o-nitrophenylsulfenyl)-2′, 3,6′-tris-N-trichloracetyl-2″-O-[dimethyl-(1,2-dimethyl-propyl)-silyl]-sisomicin

8,8 g 1,3″-Bis-N-o-nitrophenylsulfenyl)-2″-O-[dimethyl-(1,2-dimethyl-propyl)-silyl]-sisomicin in 20 ml Dichlormethan/20 ml Pyridin werden bei -15°C tropfenweise mit 7,5 ml Trichloressigsäureanhydrid versetzt und noch 10 Minuten bei Raumtemperatur weitergerührt. Nach Zugabe von 20 ml Dichlormethan wird der Ansatz zweimal mit je 20 ml $H_2O$ ausgeschüttelt, die organische Phase eingedampft und der Rückstand als Rohprodukt weiterverarbeitet. $R_F$ ($CH_2Cl_2/CH_3OH$ = 97,5/2,5) = 0,72.

Beispiel 4

1,3″-Bis-N-(o-nitrophenylsulfenyl)-2′, 3,6′-tris-N-trichloracetyl-sisomicin

Das rohe Öl von Beispiel 3 wird in 20 ml Dimethylsulfoxid gelöst, mit 2 ml einer 50 proz. KF-Lösung versetzt und 3 Stunden kräftig gerührt. Das Produkt wird mit Wasser ausgefällt, mit Wasser gewaschen und getrocknet. Das Rohprodukt wird ohne weitere Reinigung weiterverarbeitet.

13-C-NMR ($CDCl_3$):
δ = 103,60 (C-1″); 66,48 (C-3″); 55,15 (C-1);
50,60 (C-3); 79,86 (C-4); 76,18 (C-5);
89,16 (C-6); 97,74 (C-1′); 96,84 (C-4′);
149,80 (C-5); 92,78 ($CCl_3$); 162,29 und
162,11 (CO) ppm.

Beispiel 5

3″-N-(o-nitrophenylsulfenyl)-2′,3,6′-tris-N-trichloracetyl-sisomicin

Das Produkt von Beispiel 4 wird in 13 ml Dichlormethan gelöst, mit 26 ml Methanol und 5 g 2-Mercaptobenzthiazol bis zur klaren Lösung geschüttelt und 3 Tage bei 5°C stehen gelassen. Der Niederschlag wird abfiltriert, das Filtrat eingedampft und an Kieselgel chromatographiert (Laufmittel a: $CH_2Cl_2/CH_3OH$ = 95/5; b: ($CH_2Cl_2$/$CH_3OH$/20% wässriges $NH_3$ = 93/6,5/0,5).

13-C-NMR ($CDCl_3$)
δ = 103,43 (C-1″); 67,46 (C-3″); 50,85 (C-1);
50,28 (C-3); 79,44 (C-4); 76,51 (C-5);
89,29 (C-6); 97,61 (C-1′); 96,62 (C-4′);
149,50 (C-5′); 92,46 und 92,38 ($CCl_3$);
162,01 und 161,76 (CO) ppm.

Beispiel 6
(4-Nitrophenyl)-(2-trifluoracetylaminoethyl)-carbonat

30,5 g 2-Aminoethanol werden in 200 ml Acetonitril unter Eiskühlung mit 52,5 g Trifluoressigsäureanhydrid versetzt, wobei die Temperatur zwischen 5-20°C gehalten wird. Nach Abklingen der Reaktion wird eingeengt und fraktioniert. Ausbeute:

37,5 g 2-(Trifluoracetylamino)-ethanol vom Siedepunkt 130-131°C/11 mm (kristallisiert langsam durch).

17 g (0,108 mol) 2-(Trifluoracetylamino)-ethanol löst man in 210 ml Pyridin, versetzt mit 21 g Chlorameisensäure-4-nitrophenylester und lässt bei Raumtemperatur über Nacht stehen. Danach wird eingeengt, der Rückstand in Methylenchlorid aufgenommen, mit Eiswasser gewaschen und mit $Na_2SO_4$ getrocknet. Nach Abdampfen des Lösungsmittels erhält man 33 g eines hellen Öls.
IR: 1720, 1770/cm. Das Produkt enthält ca. 30% 4-Nitrophenol.

Beispiel 7
(4-Nitrophenyl)-(3-trifluoracetylaminopropyl)-carbonat

Man stellt analog Beispiel 6 aus dem 3-(Trifluoracetylamino)-propan-1-ol (Siedepunkt 120°C/3 mm; IR: 1710/cm) das aktivierte Carbonat her; IR: 1710, 1765/cm.

Beispiel 8
(4-Nitrophenyl)-(6-trifluoracetylaminohexyl-1)-carbonat

Analog Beispiel 6 erhält man aus dem 6-(Trifluoracetylamino)-hexan-1-ol (Siedepunkt 150-154°C/3,5 mm; (IR: 1710/cm) das aktivierte Carbonat als Rohprodukt; IR 1720, 1770/cm.

Beispiel 9
(4-Nitrophenyl)-[2-(2-nitrophenylsulfenyl-methyl-amino)-ethyl]-carbonat

Man legt eine Lösung von 1,4 g 2-Methylamino-ethanol in 30 ml Dioxan vor und tropft gleichzeitig unter Aufrechterhaltung von pH 8 eine Lösung von 3,8 g o-Nitrophenylsulfensäurechlorid in 10 ml Dioxan und 8,5 ml 2n-Natronlauge zu. Nach mehrstündigem Rühren bei Raumtemperatur wird im Vakuum eingeengt, der Rückstand in Essigester aufgenommen und zweimal mit Wasser gewaschen, mit $Na_2SO_4$ getrocknet und im Vakuum eingeengt. Das zurückbleibende Öl wird mit Toluol/Essigester (2:1) über 100 g Kieselgel chromatographiert und die Hauptkomponente ($R_f$: 0,29) abgetrennt. Ausbeute: 2,9 g N-(2-Hydroxyethyl)-N-methyl-o-nitrosulfensäureamid; Schmelzpunkt: 53-56°C.

456 mg dieser Verbindung und 600 mg Chlorameisensäure-p-nitrophenylester werden in 5 ml Acetonitril gelöst und unter Eiskühlung mit 300 mg Triäthylamin in 5 ml Acetonitril versetzt. Nach 1 Stunde bei Raumtemperatur wird im Vakuum eingeengt, in 30 ml Methylenchlorid aufgenommen, zweimal mit Wasser gewaschen, mit $Na_2SO_4$ getrocknet, im Vakuum eingeengt und das erhaltene orange Öl mit Toluol/Essigester (2:1) über 100 g Kieselgel chromatographiert und die Hauptfraktion abgetrennt.
Ausbeute: 250 mg oranges Öl, das langsam durchkristallisiert.
IR (KBr): 1770 $cm^{-1}$. $R_f$-Wert (Toluol/Essigester 2:1): 0,84.

Beispiel 10
(4-Nitrophenyl)-[2-(2-nitrophenylsulfenylamino)-ethyl]-carbonat

Man verfährt analog Beispiel 9; IR: 1770 $cm^{-1}$; $R_f$-Wert (Toluol/Essigester 2:1): 0,77.

Beispiel 11
1-N-(2-Aminoethyloxycarbonyl)-3,2′,6′,3″-tetra-N-(o-nitrophenylsulfenyl)-sisomicin

3,3 g 3,2′,6′,3″-Tetra-N-(o-Nitrophenylsulfenyl)-sisomicin (DE-OS 2726197) werden in 15 ml Pyridin gelöst, mit 3,5 g des Produktes aus Beispiel 6 versetzt, bei Raumtemperatur gerührt und der Verlauf der Reaktion dünnschichtchromatographisch (Kieselgel-Fertigplatten; Laufmittel: Methylenchlorid/Methanol = 95/5) verfolgt. Nach Stehen über Nacht wird im Hochvakuum eingeengt, der Rückstand in 140 ml Methylenchlorid/60 ml Methanol aufgenommen und 6 ml 4n-NaOH unter Rühren eingetropft. Nach 1 Stunde bei Raumtemperatur ist die Abspaltung beendet. Man trennt die NaOH-Phase ab und wäscht die organische Phase solange mit Wasser bis sie nicht mehr basisch reagiert. Nach dem Trocknen mit $Na_2SO_4$ wird im Vakuum eingedampft und der Rückstand zur Abtrennung einiger unpolarer Verunreinigungen an 200 g Kieselgel chromatographiert, wobei zuerst mit Methylenchlorid, anschliessend mit Methylenchlorid/Methanol (5:1) eluiert wird.
Ausbeute: 2,2 g oranges Festprodukt;
$R_f$-Wert (Methylenchlorid/Methanol 5:1): 0,4.

Beispiel 12
1-N-(3-Aminopropyloxycarbonyl)-3,2′,6′,3″-tetra-N-(o-nitrophenylsulfenyl)-sisomicin

Man verfährt entsprechend Beispiel 11 unter Verwendung des aktivierten Carbonats aus Beispiel 7 und erhält das Reaktionsprodukt in 55% Ausbeute. $R_f$-Wert (Methylenchlorid/Methanol 5:1): 0,42.

Beispiel 13
1-N-(6-Aminohexyloxycarbonyl)-3,2′,6′,3″-tetra-N-(o-nitrophenylsulfenyl)-sisomicin

Man verfährt entsprechend Beispiel 11 unter Verwendung des Carbonats aus Beispiel 8 und erhält das Sisomicinderivat in 52% Ausbeute.
$R_f$-Wert (Methylenchlorid/Methanol 5:1): 0,5.

Beispiel 14
1-N-(2-Aminoethyloxycarbonyl)-3″-N-(o-nitrophenylsulfenyl)-3,2′,6′-tris-N-(trichloracetyl)-sisomicin

Zu einer Lösung von 1,04 g des Produktes aus Beispiel 5 in 5 ml Pyridin werden 500 mg des Produktes aus Beispiel 10 gefügt und bei Raumtemperatur gerührt. Nach 2 Stunden ist dünnschichtchromatographisch (Kieselgel-Fertigplatten, Laufmittel = Methylenchlorid/Methanol/20% aq. $NH_3$ = 930/65/5) keine Ausgangsverbindung mehr nachweisbar. Man dampft im Hochvakuum ein, nimmt in Methylenchlorid auf, wäscht mit Wasser, trocknet mit $Na_2SO_4$ und engt erneut ein. Zur Abspaltung der NPS-Gruppe von der primären Aminogruppe nimmt man den Rückstand in 3 ml Methanol/2 ml Methylenchlorid auf, versetzt mit 170 mg 2-Mercaptobenzthiazol und lässt 1 Tag bei Raumtemperatur stehen. Danach wird eingeengt und das polare Spaltprodukt von einigen unpolaren Komponenten durch Chromatographie an 70 g Kieselgel (Laufmittel wie oben) gereinigt. Ausbeute: 577 mg oranges Festprodukt. $R_f$-Wert (Laufmittel wie oben): 0,15.

Beispiel 15
1-N-(2-Methylaminoethyloxycarbonyl)-3″-N-(o-nitrophenylsulfenyl)-3″,6′-tris-N-(trichloracetyl)-sisomicin

Man verfährt analog Beispiel 14 und erhält das Sisomicinderivat in 45% Ausbeute. $R_f$-Wert (Methylenchlorid/Methanol/20% $NH_3$ = 930/65/5): 0,17.

Beispiel 16
1-N-[2-(1,3-Dihydroxypropyl-2-amino)-ethyloxycarbonyl]-sisomicin

115 mg des Produktes aus Beispiel 11 werden in 2,5 ml Methanol/0,4 ml Methylenchlorid/0,5 ml Wasser gelöst, mit Essigsäure auf pH 6 eingestellt und mit 20 mg Dihydroxyaceton versetzt. Man rührt 15 Minuten bei Raumtemperatur und gibt dann 15 mg $NaBCNH_3$ zu. Nach weiteren 30 Minuten lässt sich dünnschichtchromatographisch ein neues Produkt nachweisen (DC: Kieselgel; Methylenchlorid/Methanol = 5:1; $R_f$-Wert = 0,53), das durch Versetzen mit 5 ml Essigester, Waschen mit 2×5 ml Wasser, Trocknen mit $Na_2SO_4$ und Einengen des Filtrats im Vakuum isoliert wird. Die Abspaltung der o-Nitrophenylsulfenyl-Schutzgruppen erfolgt durch Lösen des Rückstandes in 0,6 ml Methylenchlorid, Versetzen mit 1,6 ml einer Lösung von 8,5 g 2-Mercaptobenzthiazol in 30 ml Methanol/50 ml Methylenchlorid und Ansäuern mit verdünnter Salzsäure bis zur Farbaufhellung. Aus der Spaltlösung wird das Sisomicinderivat mit 1 ml Wasser extrahiert, die wässrige Phase mit 2×0,5 ml Methylenchlorid gewaschen und mit dem basischen Ionenaustauscher Lewatit MP 500 ($OH^-$) basisch gestellt. Man dampft ein und chromatographiert zur Entfernung weniger Verunreinigungen über 5 g Kieselgel mit Methylenchlorid/Methanol/20% $NH_3$ (2:4:1) als Laufmittel), Ausbeute = 29 mg. $R_f$-Wert (Kieselgel, Methylenchlorid/Methanol/konz. $NH_3$ = 2:2:1): 0,44.

Die in den folgenden Ausführungsbeispielen angegebenen $R_f$-Werte wurden auf DC-Fertigplatten Kieselgel 60 $F_{254}$ der Firma Merck, Darmstadt, ermittelt. Wenn nicht anders angegeben, wurde das Laufmittelsystem Methylenchlorid/Methanol/20% wässrige $NH_3$-Lösung (2:4:1) benutzt. Zur Anfärbung der Substanzen wurden die Platten in einer Jodkammer bedampft.

Zur chromatographischen Reinigung diente Kieselgel 60 (0,063–0,2 mm) der Firma Merck, Darmstadt.

Beispiel 17
1-N-[2-[(S)-2,3-Dihydroxypropylamino]-ethyloxycarbonyl]-sisomicin

Man verfährt wie in Beispiel 16, verwendet jedoch 2,3-O-Isopropyliden-D-glycerinaldehyd als Carbonylkomponente und erhitzt 2½ Stunden unter Rückfluss. Nach Abspaltung der o-Nitrophenylsulfenylschutzgruppen bleibt die saure Reaktionslösung zur Entfernung der Isopropylidenschutzgruppe über Nacht stehen. Bei der anschliessenden chromatographischen Reinigung an Kieselgel mit Methylenchlorid/Methanol/20% $NH_3$ (2:4:1) als Elutionsmittel erhält man 17 mg mit einem $R_f$-Wert: 0,31; identisch mit dem Produkt aus Beispiel 31 f.

Beispiel 18
1-N-[2-(1-Hydroxypropyl-2-amino)-ethyloxycarbonyl]-sisomicin

Man verfährt wie in Beispiel 16, verwendet jedoch Hydroxyaceton als Carbonylreagens. Ausbeute: 22 mg; $R_f$-Wert: 0,28.

Beispiel 19
1-N-[2-[(S,R)-2,3,4-Trihydroxybutylamino]-ethyloxycarbonyl]-sisomicin

Man verfährt wie in Beispiel 16, verwendet jedoch D-Erythrose als Carbonylreagens. Ausbeute: 15 mg; $R_f$-Wert: 0,28.

Beispiel 20
1-N-[2-(4-Hydroxy-but-2-enylamino)-ethyloxycarbnyl]-sisomicin

Man verfährt wie in Beispiel 16, verwendet jedoch 4-(2-Tetrahydropyranyl)-but-2-en-1-al (e. J. Corey u. J.W. Suggs Tetrahedron Letters 1975, 2647) als Carbonylreagens. Nach Abspaltung der o-Nitrophenylsulfenylschutzgruppen mit 2-Mercaptobenzthiazol/HCl bleibt die saure Lösung noch 2 Stunden bei Raumtemperatur stehen, um zusätzlich die Tetrahydropyranylschutzgruppe zu entfernen. Erst dann wird mit dem anionischen Austauscher Lewatit MP 500 ($OH^-$) behandelt und das Reaktionsprodukt gereinigt. Ausbeute: 12 mg; $R_f$-Wert: 0,40.

Beispiel 21
1-N-[2-(4-Hydroxybut-2-ylamino)-ethyloxycarbonyl]-sisomicin

Man verfährt wie in Beispiel 16, verwendet jedoch 4-Hydroxybutan-2-on als Carbonylkomponente. Ausbeute: 12 mg; $R_f$-Wert: 0,44.

Beispiel 22
1-N-[2-[(S,S,R)-2,3,4,5-Tetrahydroxypentylamino]-ethyloxycarbonyl]-sisomicin

Man verfährt wie in Beispiel 16, verwendet aber D-Ribose als Carbonylkomponente und erhitzt 30

Minuten unter Rückfluss. Ausbeute: 13 mg; $R_f$-Wert: 0,28.

Beispiel 23
1-N-[2-[(R,S,R)-2,3,4,5-Tetrahydroxypentylamino]-ethyloxycarbonyl]-sisomicin

Man verfährt wie in Beispiel 16, verwendet jedoch D-Arabinose und erhitzt 30 Minuten unter Rückfluss. Ausbeute: 20 mg; $R_f$-Wert: 0,46 (Methylenchlorid/Methanol/konz. $NH_3$ = 2:2:1) (Sisomicin: 0,51)

Beispiel 24
1-N-[2-[(S,R,S)-2,3,4,5-Tetrahydroxypentylamino]-ethyloxycarbonyl]-sisomicin

115 mg des Produktes aus Beispiel 11 werden in

a) 2,5 ml Tetrahydrofuran/0,5 ml Wasser
b) 2,5 ml Ethanol/0,5 ml Wasser
c) 2,5 ml Isopropanol/0,5 ml Wasser
d) 2,5 ml tert.-Butanol/0,5 ml Wasser

gelöst, mit Essigsäure auf pH 6 eingestellt, mit 20 mg L-Arabinose versetzt und 30 Minuten bei Raumtemperatur gerührt. Danach gibt man 15 mg $NaBCNH_3$ zu, rührt 4 Stunden bei Raumtemperatur, engt ein und behandelt mit Methylenchlorid/Wasser. Die Methylenchloridphase wird mit $Na_2SO_4$ getrocknet, eingeengt und zur Isolierung der orangen Hauptkomponente an Kieselgel mit Methylenchlorid/Methanol (5:1) als Laufmittel chromatographiert. Die weite Aufarbeitung erfolgt wie in Beispiel 16 und man erhält folgende Ausbeuten) a) 9 mg, b) 11 mg, c) 14 mg, d) 13 mg.
$R_f$-Wert: 0,44 (Methylenchlorid/Methanol/konz. $NH_3$ = 2:2:1).

Beispiel 25
1-N-[2-(4,5-Dihydroxy-pentylamino)-ethyloxycarbonyl]-sisomicin

a) 4,5-Cyclohexylidendioxy-pentan-1-ol:

12 g 1,2,5-Trihydroxypentan werden mit 9,8 g Cyclohexanon und 250 mg p-Toluolsulfonsäure in 50 ml Toluol 7 Stunden am Wasserabscheider erhitzt. Anschliessend wird mit 5% $K_2CO_3$-Lösung und Wasser gewaschen, mit $Na_2SO_4$ getrocknet und destilliert. Ausbeute: 9 g vom Siedepunkt 128°/8 mm.

b) 4,5-Cyclohexylidendioxy-pentan-1-ol:

Die Oxydation von 4,5-Cyclohexylidendioxy-pentan-1-ol mit Pyridiniumchlorochromat nach E.J. Corey u.a., Tetrahedron Letters 1975, 2647 liefert den Aldehyd.

c) 1-N-[2-(4,5-Dihydroxy-pentylamino)-ethyloxycarbonyl]-sisomicin:

Man verfährt wie in Beispiel 16 unter Verwendung von 4,5-Cyclohexylidendioxy-pentan-1-al als Carbonylreagens. Nach Abspaltung der o-Nitrophenylsulfenylgruppen wird die Cyclohexyliden-Schutzgruppe durch Stehenlassen der sauren Lösung abgespalten.
Ausbeute: 13 mg; $R_f$-Wert: 0,45.

Beispiel 26
1-N-[2[(S,R,R,R)-2,3,4,5,6-Pentahydroxy-hexylamino]-ethyloxycarbonyl]-sisomicin

Man verfährt wie in Beispiel 16 unter Verwendung von D-Glucose als Carbonylreagens mit Ausdehnung der Reaktionsdauer auf 16 Stunden. $R_f$-Wert: 0,35 (Methylenchlorid/Methanol/konz. $NH_3$ = 2:2:1).

Beispiel 27
1-N-[3-[(S)-2,3-Dihydroxypropylamino]-propyloxycarbonyl]-sisomicin

Man verfährt wie in Beispiel 17 unter Verwendung des Zwischenproduktes aus Beispiel 12. Ausbeute: 15 mg; $R_f$-Wert: 0,32.

Beispiel 28
1-N-[6-[(S)-2,3-Dihydroxypropylamino]-hexyloxycarbonyl]-sisomicin

Man verfährt wie in Beispiel 17 unter Verwendung des Zwischenproduktes aus Beispiel 13. Ausbeute: 12 mg; $R_f$-Wert: 0,40.

Beispiel 29
1-N-[3-(1,3-Dihydroxyprop-2-ylamino)-propyloxycarbonyl]-sisomicin

Man verfährt wie in Beispiel 16 unter Verwendung des Zwischenproduktes aus Beispiel 12. Ausbeute: 10 mg; $R_f$-Wert: 0,46.

Beispiel 30
1-N-[2[(S)-2,3-Dihydroxypropylamino]-ethyloxycarbonyl]-sisomicin

Man verfährt wie in Beispiel 17 unter Verwendung des Zwischenproduktes aus Beispiel 14. Nach der Isolierung des Reaktionsproduktes werden die Schutzgruppen in folgender Weise entfernt:

a) Abspaltung der Trichloracetylgruppen in Methanol mit 4n-KOH;

b) Abspaltung der o-Nitrophenylsulfenylgruppen mit 2-Mercaptobenzthiazol/HCl in Methanol/Methylenchlorid;

c) Abspaltung der Cyclohexylidengruppe durch Stehenlassen der sauren Reaktionslösung bei Raumtemperatur.

Nach Behandlung mit Lewatit MP 500 ($OH^-$) und chromatographischer Reinigung erhält man 12 mg vom $R_f$-Wert: 0,31.

Beispiel 31
1-N-{2-[(S)-2,3-Dihydroxypropylamino]-ethyloxycarbonyl]-sisomicin

a) 1,2-5,6-Dicyclohexyliden-mannit

1,35 Liter Cyclohexanon (über $K_2CO_3$ getrocknet und destilliert) werden portionsweise mit 270 g $ZnCl_2$ (geschmolzen und zerrieben) in ca. 30–60 Min. versetzt, wobei sich die Temperatur zwischen 25° und 40° hält. Diese $ZnCl_2$-Lösung wird unter Feuchtigkeitsausschluss zu 170 g (0,93 mol) Mannit gegeben und 90 Min. bei 35–37°

gerührt, bis der Mannit gelöst ist. Dann wird das Reaktionsgemisch rasch und unter gutem Rühren in eine vorbereitete Lösung von 340 g $K_2CO_3$/340 ml Wasser, die mit 1,35 Liter Ether bedeckt ist, eingerührt, nach 30 Min. von $ZnCO_3$ abgesaugt, mit Ether gewaschen und im Vakuum eingeengt. Man erhält 281 g eines wachsartigen Rückstandes, der in 300 ml Toluol heiss gelöst wird. Das Filtrat dieser Lösung wird nach dem Abkühlen mit 1 Liter Petrolether versetzt, der gallertige Niederschlag abgesaugt (Fritte) und nochmals mit 3×350 ml Petrolether verrührt. Nach dem Trocknen bei 50° im Vakuum über Paraffinöl erhält man 188 g (59%) Dicyclohexyliden-mannit; Schmp. 101-102°.
NMR ($CDCl_3$): δ = 1,56 (s, breit; 20 Cyclohexyliden-H), 2,93 (d, J = 7 Hz; 2OH), 3,5-4,4 ppm (m; 8H).

b) Cyclohexyliden-D-glycerinaldehyd

55,4 g (0,16 mol) 1,2-5,6-Bicyclohexyliden-mannit in 800 ml Aceton gibt man zu 735 ml einer Pufferlösung (hergest. aus 6,8 g $KH_2PO_4$ in 500 ml Wasser +56 ml 0,1n-NaOH; mit Wasser auf 1 Liter aufgefüllt), bringt den pH-Wert durch Zugabe von 2 ml 2n-NaOH auf 7-8 und versetzt bei Raumtemperatur mit 35,5 g (0,166 mol) $NaIO_4$, wobei die Temperatur auf 31° ansteigt. Nach 70 Min. wird i.Vak. eingeengt, der Rückstand in 500 ml Wasser aufgenommen und mit 7×150 ml Methylenchlorid extrahiert. Man erhält 47,2 g (85%) rohen Cyclohexylidenglycerinaldehyd, der wegen seiner Polymerisationsneigung sofort umgesetzt wird. Eine Destillation von 11,7 g Rohprodukt lieferte 8 g reinen Aldehyd vom Sdp. 102°/1 mm.
NMR ($CDCl_3$): δ = 1,62 (s, breit; 10 Cyclohexyliden-H), 3,95-4,55 (m; $-CH_2-CH<$), 9,77 ppm (d, J = 2 Hz; CHO).

c) D-2-(1,4-Dioxa-spiro[4,5]decan-2-yl-methylamino)-ethanol

102 g (1,68 mol) Aminoethanol werden in 800 ml Methanol gelöst und mit methanolischer Salzsäure auf pH 7,1 eingestellt. Nach Zugabe von 47,2 g (0,27 mol) Cyclohexyliden-D-glycerinaldehyd (pH fällt auf 4,3) wird mit 10,8 g (0,17 mol) $NaBCNH_3$ versetzt (pH steigt auf 6,9-7,1). Man rührt 3 Stdn. bei Raumtemperatur nach und lässt über Nacht stehen. Die Suspension wird eingeengt, in 500 ml Wasser gelöst und mit 21 ml verd. HCl (1:1) auf pH 6 eingestellt, so dass die nichtbasischen Anteile mit Ether extrahiert werden können. Anschliessend wird die wässrige Phase mit 65 ml 2n-NaOH auf pH 8,3 eingestellt und mit 13×150 ml Methylenchlorid extrahiert, mit Wasser gewaschen, mit $Na_2SO_4$ getrocknet und i.Vak. eingeengt.
Ausbeute: 36,6 g Öl (63%).
$R_f$-Wert: 0,57 (Methylenchlorid/Methanol/17% $NH_3$ = 15:2:0,1).

d) D-N-(2-Nitrophenylsulfenyl)-2-(1,4-dioxa-spiro[4,5]decan-2-yl-methylamino)-ethanol

Eine Lösung von 42,4 g D-2-(1,4-Dioxa-spiro[4,5]decan-2-yl-methylamino)-ethanol (0,2 mol) in 375 ml Dioxan wird unter pH-Kontrolle gleichzeitig mit einer Lösung von 37,8 g (0,2 mol) o-Nitrophenylsulfenylchlorid in 190 ml Dioxan und 144 ml 2n-NaOH versetzt, so dass der pH-Wert >8 bleibt. Danach wird eingeengt, in 400 ml Methylenchlorid aufgenommen, mit Wasser gewaschen, mit $Na_2SO_4$ getrocknet und i.Vak. eingeengt. Man erhält 84 g eines Rohprodukts, das zur Reinigung in Toluol gelöst und über eine Säule mit 1,7 kg Kieselgel filtriert wurde. Als Elutionsmitel dienten

a) Toluol zum Herauswaschen unpolarer Verunreinigungen

b) Toluol/Essigester-Gemische mit steigendem Essigestergehalt (9:1) → (8:2) → (7:3).
Ausbeute: 65,1 g (88%) oranges Öl;
$R_f$-Wert: 0,3 (Toluol/Essigsäureethylester = 2:1).
NMR ($CDCl_3$): δ = 1,4 (s. breit; 10 Cyclohexyliden-H), 3,1-4,7 (m, 9 aliphatische H, 1 OH), 7,2-8,4 ppm (m, 4 aromatische H).

e) D-(4-Nitrophenyl)-[N-(2-nitrophenylsulfenyl)-2-(1,4-dioxaspiro[4,5]decan-2-yl-methylamino)-ethyl]-carbonat

50,2 g (0,136 mol) der Stufe d) werden in 710 ml absol. Pyridin gelöst und unter Eiskühlung portionsweise mit 27,5 g (0,137 mol) Chlorameisensäure-p-nitrophenylester versetzt, so dass sich die Temperatur bei 20-21° hält. Man rührt 90 Min. bei Raumtemperatur und 4 Stdn. bei 40°. Danach filtriert man ca. 4 g ungelösten Chlorameisensäure-p-nitrophenylester ab, engt ein, löst den Rückstand in Methylenchlorid, wäscht dreimal mit Eiswasser, trocknet mit $Na_2SO_4$, filtriert und dampft i.Vak. ein. Den Rückstand (79,6 g) löst man in wenig Methylenchlorid und filtriert über 1,4 kg Kieselgel mit Methylenchlorid als Elutionsmittel.
Ausbeute: 37 g oranges Öl (51%);
$R_f$-Wert: 0,4 (Methylenchlorid).
NMR ($CDCl_3$): δ = 1,6 (s, breit; 10 Cyclohexyliden-H), 3,2-4,7 (m, 9 aliphatische H), 7,2-8,5 ppm (m mit dem AB-System des 4- Nitrophenylesters; 8 arom. H).

f) 1-N-[2-[(S)-2,3-Dihydroxypropylamino]-ethyloxycarbonyl)]-sisomicin

Eine Lösung von 39 g (72%ig, $3,7 \times 10^{-2}$ mol) des Produktes aus Beispiel 5 in 200 ml Pyridin wird mit 20,7 g ($3,9 \times 10^{-2}$ mol) der Stufe e) bei Raumtemperatur versetzt und über Nacht stehengelassen. Man dampft i.H. Vak. ein (62,3 g) oranges Öl) und filtriert den Rückstand über eine Säule mit 500 g Kieselgel, wobei nacheinander mit a) Methylenchlorid 4-Nitrophenol und einige Verunreinigungen und b) mit Methylenchlorid/Methanol/20% $NH_3$ (930:65:5), das Reaktionsprodukt eluiert werden. Man erhält so 38 g eines orangen Schaums, den man zur Abspaltung der Trichloracetylgruppen in 785 ml Methanol löst, mit 250 ml 4n-KOH versetzt und über Nacht bei Raumtemperatur stehen lässt. Die alkalische Lösung wird im Vakuum bis auf ca. 100 ml eingeengt und die wässrige Phase von dem ungelösten orangen

Rückstand abdekantiert, den man in Methylenchlorid aufnimmt und mit Wasser neutral wäscht. Nach dem Trocknen mit $Na_2SO_4$ und Einengen isoliert man 23 g eines Zwischenproduktes, das in 60 ml Methylenchlorid aufgenommen, mit einer Lösung von 35,5 g (0,21 mol) 2-Mercaptobenzthiazol in 125 ml Methanol/210 ml Methylenchlorid versetzt und mit wässriger HCl (1:1) bis zum Farbumschlag nach Gelb angesäuert wird. Das Sisomicinderivat wird mit 2×150 ml Wasser extrahiert, die wässrige Phase mit 3×50 ml $CH_2Cl_2$ gewaschen und die saure Lösung zur Abspaltung der Cyclohexylidengruppe 2 Tage bei Raumtemperatur stehen gelassen, anschliessend mit Lewatit MP 500 ($OH^-$) basisch gestellt und im Vakuum eingeengt.

Ausbeute: 16 g eines Rohproduktes, aus dem nach chromatographischer Reinigung an Kieselgel mit Methylenchlorid/Methanol/20% $NH_3$ (2:4:1) als Elutionsmittel 11,4 g (51%) reines Sisomicinderivat erhalten wurden. $R_f$-Wert: 0,31 (Sisomicin: 0,26).
$^{13}$C-NMR ($D_2O$/Dioxan): $\delta$ = 52,08 (C-1); 35,62 (C-2);
51,52 (C-3); 84,59 (C-4); 75,61 (C-5); 81,78 (C-6); 158,71 (CO); 68,27 ($CH_2$, $\alpha$ zu COO); 48,04 ($CH_2$, $\beta$ zu COO);
49,85 ($CH_2$, $\alpha$ zu NH); 71,25 (CHOH), 64,93 ($CH_2OH$) ppm.

Beispiel 32
1-N-[2-(2,3-Dihydroxypropylamino)-ethyloxycarbonyl]-sisomicin

a) D,L-2-(1,4-Dioxa-spiro[4,5]decan-2yl-methylamino)-ethanol

Man erhitzt 38 g (0,2 mol) 2-Chlormethyl-1,4-dioxaspiro[4,5]decan (Deutsche Auslegeschrift 1 593 797) in 150 ml Aminoethanol mit 50 g wasserfreiem zerriebenem $Na_2CO_3$ 18 Stunden unter Rückfluss. Das ungelöste Salz wird über eine Fritte abgesaugt, überschüssiges Aminoethanol im Vakuum abdestilliert, der Rückstand mit Methylenchlorid verrührt und nochmals abgesaugt. Nach Einengen des Filtrats erhält man ein Öl, das zur Abtrennung des stark polaren Aminoethanolrestes über 220 g Kieselgel filtriert wurde (Methylenchlorid/Methanol/17% $NH_3$ = 15:2:0,1 als Elutionsmittel). Ausbeute: 32 g (74%) Öl.
Bei der Reinigung durch Destillation (SDp. 145°C/1,3 mm) wird eine Ausbeute von 52% erreicht. $R_f$-Wert: 0,57 (Methylenchlorid/Methanol/17% $NH_3$ = 15:2:0,1).

b) 1-N-[2-(2,3-Dihydroxypropylamino)-ethyloxycarbonyl]-sisomicin

Man verfährt wie in Beispiel 31, Stufen d)-f) beschrieben und isoliert das Sisomicinderivat mit gleichem $R_f$-Wert: in einer Ausbeute von 48%.

Beispiel 33
1-N-[2-[(S)-2,3-Cyclohexylidendioxypropylamino]-ethyloxycarbonyl]-sisomicin

Man verfährt wie in Beispiel 31-f mit dem Unterschied, dass nach der o-Nitrophenylsulfenylgruppen-Abspaltung mit 2-Mercaptobenzthiazol/HCl sofort mit Lewatit MP 500 ($OH^-$) basisch gestellt wird, um die Hydrolyse der Cyclohexylidengruppe zu vermeiden. Anschliessend wird eingeengt und chromatographisch gereinigt.

$R_f$-Wert: 0,71 (Methylenchlorid/Methanol/20% $NH_3$ = 2:4:1)
$^{13}$C-NMR ($D_2O$): $\delta$ = 51,45 (C-1); 31,86 (C-2); 51,16 (C-3); 81,22 (C-4); 74,75 (C-5); 80,96 (C-6); 157,59 (CO); 61,50 ($CH_2$, $\alpha$ zu COO); 47,26 ($CH_2$, $\beta$ zu COO); 49,66 ($CH_2$ $\alpha$ zu NH); 66,99 (CH-O), 66,90 ($CH_2O$); 112,71 (C-1 des Cyclohexylidenrestes); 34,61 und 35,86 (C-2 und C-6 des Cyclohexylidenrestes); 24,14 und 24,40 (C-3 und C-5 des Cyclohexylidenrestes); 23,88 (C-4 des Cyclohexylidenrestes) ppm.

Beispiel 34
1-N-[2-[(S)-2,3-Isopropylidendioxypropylamino]-ethyloxycarbonyl]-sisomicin

a) D-2-(2,3-Isopropylidendioxypropylamino)-ethanol

Man verfährt analog Beispiel 31 c), wobei für die reduktive Alkylierung Isopropyliden-D-glycerinaldehyd verwendet wird. Ausbeute: 62%; $R_f$-Wert: 0,45 (Methylenchlorid/Methanol/17% $NH_3$ = 15:2:0,1).

b) D-N-(o-Nitrophenylsulfenyl)-2-(2,3-isopropylidendioxypropylamino)-ethanol

Man verfährt analog Beispiel 31 d), wobei man von dem Produkt aus Beispiel 34 a) ausgeht. Ausbeute: 79%.

c) D-(4-Nitrophenyl)-[N-(o-nitrophenylsulfenyl)-2-(2,3-isopropylidendioxypropylamino)-ethyl]carbonat

Man verfährt wie in Beispiel 31 e), wobei man das Produkt aus Beispiel 34 b) verwendet. Ausbeute: 55%.

d) 1-N-[2-[(S)-2,3-Isopropylidendioxypropylamino]-ethyloxycarbonyl]-sisomicin

Man verfährt entsprechend Beispiel 33 unter Verwendung des Produktes aus Beispiel 34 c) als Acylierungsmittel. $R_f$-Wert: 0,69.

**Patentansprüche**

1. Verbindungen der Formel

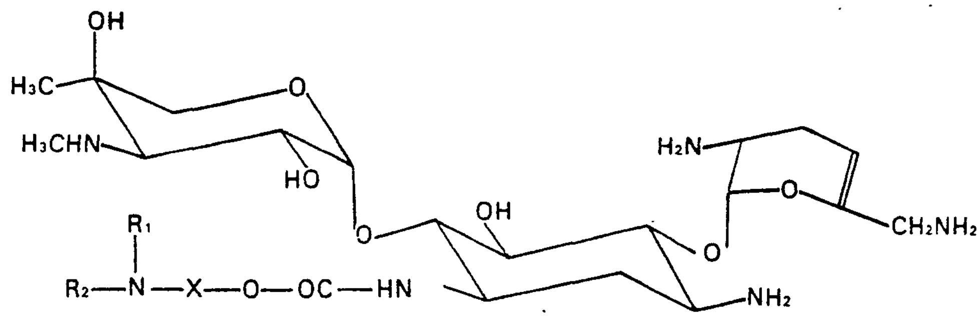

worin X einen geradkettigen, verzweigten oder cyclischen gesättigten oder ungesättigten aliphatischen Rest mit 2 bis 10 C-Atomen,
$R_1$ Wasserstoff, $C_1$-$C_4$-Alkyl oder Benzyl und
$R_2$ einen Rest der Formel

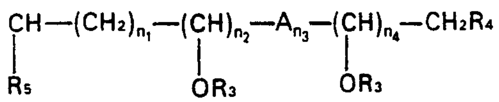

bedeuten, wobei
A für

$$—CH{=}CH—, \quad \left[— CH_{(2-n_5)} —(CH_2OR_3)_{n_5}\right]$$

$R_3$ für Wasserstoff, Triarylmethyl, Alkyl oder Acyl, oder 2 Reste $R_3$ gemeinsam Alkyliden
$R_4$ für Wasserstoff oder $OR_3$,
$R_5$ für Wasserstoff oder gegebenenfalls durch 1–3 Hydroxygruppen substituiertes $C_1$-$C_4$-Alkyl
$n_1$ für 0, 1, 2 oder 3,
$n_2$ für 0, 1, 2, 3, 4 oder 5 und
$n_3$, $n_4$ und $n_5$ unabhängig voneinander für 0, 1 oder 2 stehen, wobei die Summe aus $n_1$, $n_2$, $n_3$ und $n_4$ Werte von 1 bis 5 annimmt und die Gesamtzahl der $OR_3$ Gruppen 1 bis 6 beträgt.

2. Verbindungen gemäss Anspruch 1, worin X für einen $C_2$ bis $C_6$-Alkylenrest und $R_1$ für Wasserstoff stehen.

3. Verbindungen gemäss Anspruch 1, worin $R_3$ für Wasserstoff steht.

4. Verfahren zur Herstellung von Verbindungen gemäss Anspruch 1, in denen $R_1$ Wasserstoff bedeutet, dadurch gekennzeichnet, dass man eine Verbindung der Formel

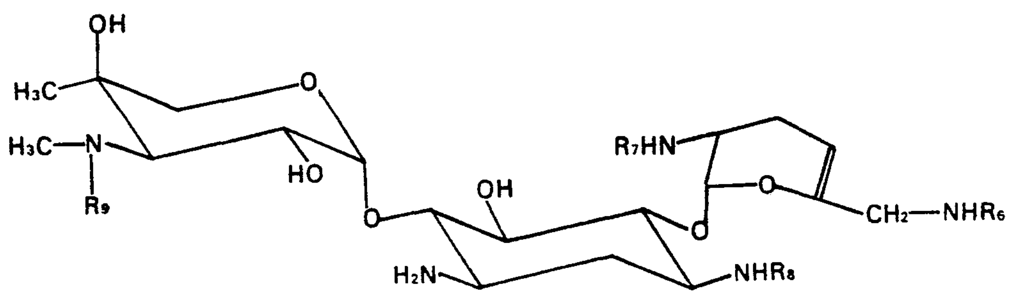

worin
$R_6$, $R_7$ und $R_8$ für -CO-$R_{10}$ oder -S-$R_{11}$ und
$R_9$ für -S-$R_{11}$ stehen,
wobei $R_{10}$ einen Rest der Formeln

$—CHal_3$, $—(CH_2)_{n_6}B$, $—O—E$,

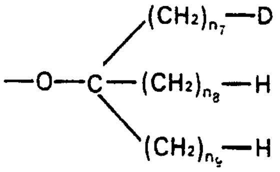

oder

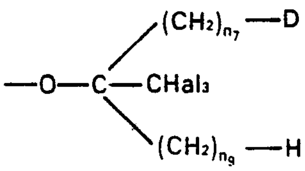

B und D Wasserstoff oder gegebenenfalls substituiertes Phenyl,
E gegebenenfalls substituiertes Phenyl,
$n_6$ eine Zahl von 0–5,
$n_7$, $n_8$ und $n_9$ eine Zahl von 0–5,
Hal Fluor, Chlor oder Brom und
$R_{11}$ gegebenenfalls substituiertes Phenyl, Di- oder Triphenylmethyl
bedeuten,
in an sich bekannter Weise mit einem Acylierungsmittel der Formel

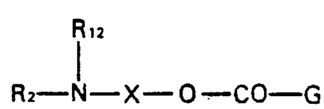

worin
$R_2$ und X die im Anspruch 1 angegebene Bedeutung haben und

G für eine Abgangsgruppe, vorzugsweise für Halogen wie Chlor und Brom, Azido oder gegebenenfalls substituiertes Phenoxy oder einen Rest der Formel

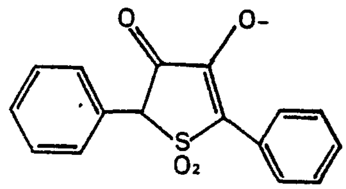

und $R_{12}$ für -CO-$R_{10}$ oder -S-$R_{11}$ stehen, umsetzt, die Schutzgruppen $R_6$, $R_7$, $R_8$, $R_9$ und $R_{12}$ sowie gegebenenfalls Alkylidengruppen, die 2 Reste $R_3$ gemeinsam bilden, in üblicher Weise abspaltet und die resultierenden Verbindungen gegebenenfalls in ihre pharmazeutisch verwendbaren Salze überführt.

5. Verfahren zur Herstellung von Verbindungen gemäss Anspruch 1, dadurch gekennzeichnet, dass man Verbindungen der Formel

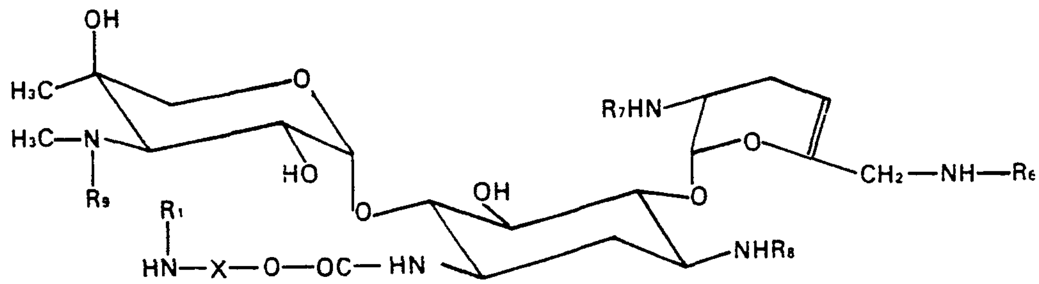

worin $R_1$, $R_6$, $R_7$, $R_8$, $R_9$ und X die in Anspruch 4 angegebene Bedeutung haben, mit Carbonylverbindungen der Formel

$$R_5-\underset{\underset{O}{\|}}{C}-(CH_2)_{n_1}-(\underset{\underset{OR_3}{|}}{CH})_{n_2}-A_{n_3}-(\underset{\underset{OR_3}{|}}{CH})_{n_4}-CH_2-R_4$$

worin $R_3$, $R_4$, $R_5$, A, $n_1$, $n_2$, $n_3$ und $n_4$ die in Anspruch 1 genannte Bedeutung haben, in Gegenwart eines Wasserstoffdonorreduktionsmittels umsetzt und anschliessend die Schutzgruppen $R_6$, $R_7$, $R_8$ und $R_9$ sowie gegebenenfalls Alkylidengruppen, die 2 Reste $R_3$ gemeinsam bilden, abspaltet und die erhaltenen Produkte gegebenenfalls in ihre pharmazeutisch verwendbaren Salze überführt.

6. Arzneimittel, gekennzeichnet durch einen Gehalt an einer Verbindung gemäss Anspruch 1 bis 3.

**Claims**

1. Compounds of the formula

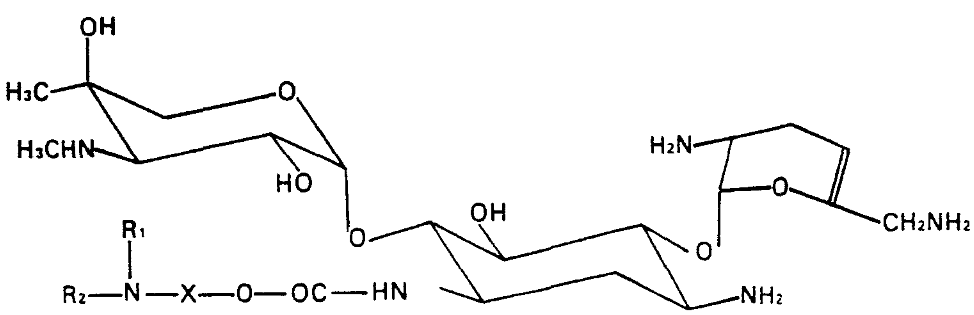

wherein
X denotes a straight-chain, branched or cyclic saturated or unsaturated aliphatic radical with 2 to 10 C atoms,
$R_1$ denotes hydrogen, $C_1$-$C_4$-alkyl or benzyl and
$R_2$ denotes a radical of the formula

$$\underset{\underset{R_5}{|}}{CH}-(CH_2)_{n_1}-(\underset{\underset{OR_3}{|}}{CH})_{n_2}-A_{n_3}-(\underset{\underset{OR_3}{|}}{CH})_{n_4}-CH_2R_4$$

wherein
A represents

$$-CH{=}CH-,\ \text{or}\ \left[-CH_{(2-n_5)}-(CH_2OR_3)_{n_5}\right]$$

$R_3$ represents hydrogen, triarylmethyl, alkyl or acyl, or 2 radicals $R_3$ together represent alkylidene,

$R_4$ represents hydrogen or $OR_3$,
$R_5$ represents hydrogen or $C_1$-$C_4$-alkyl which is optionally substituted by 1-3 hydroxyl groups
$n_1$ represents 0, 1, 2 or 3,
$n_2$ represents 0, 1, 2, 3, 4 or 5 and
$n_3$, $n_4$ and $n_5$ independently of one another represent 0, 1 or 2,
the sum of $n_1$, $n_2$, $n_3$ and $n_4$ assuming values of 1 to 5 and the total number of $OR_3$ groups being 1 to 6.

2. Compounds according to Claim 1,
wherein
X represents a $C_2$ to $C_6$-alkylene radical and
$R_1$ represents hydrogen.

3. Compounds according to Claim 1,
wherein
$R_1$ represents hydrogen.

4. Process for the preparation of compounds according to Claim 1 in which $R_1$ denotes hydrogen, characterised in that a compound of the formula

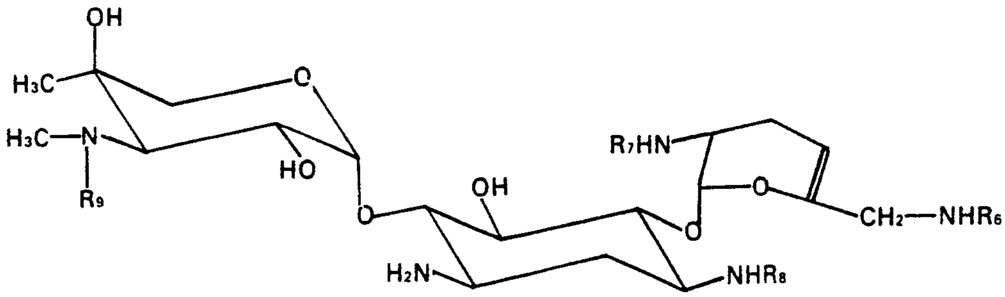

wherein
$R_6$, $R_7$ and $R_8$ represent -CO-$R_{10}$ or -S-$R_{11}$ and
$R_9$ represents -S-$R_{11}$,
wherein
$R_{10}$ denotes a radical of the formulae

$-CHal_3$, $-(CH_2)_{n_6}B$, $-O-E$,

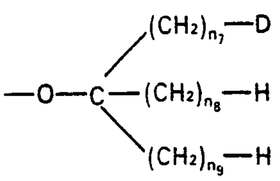

or

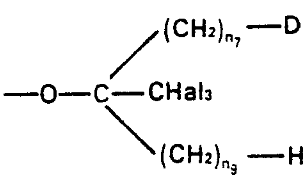

B and D denote hydrogen or optionally substituted phenyl,
E denotes optionally substituted phenyl,
$n_6$ denotes a number from 0 to 5,
$n_7$, $n_8$ and $n_9$ denote a number from 0 to 5,
Hal denotes fluorine, chlorine or bromine and
$R_{11}$ denotes optionally substituted phenyl or di- or tri-phenylmethyl,

is reacted with an acylating agent of the formula

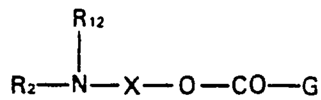

wherein
$R_2$ and X have the meaning indicated in Claim 1,
G represents a leaving group, preferably halogen, such as chlorine and bromine, azido or optionally substituted phenoxy, or a radical of the formula

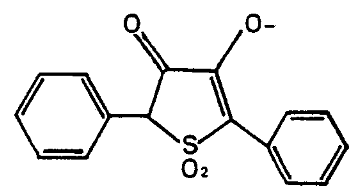

and
$R_{12}$ represents -CO-$R_{10}$ or -S-$R_{11}$,
in a manner which is in itself known, the protective groups $R_6$, $R_7$, $R_8$ $R_9$ and $R_{12}$ and, if appropriate, alkylidene groups which 2 radicals $R_3$ form together, are split off in the customary manner and the resulting compounds are optionally converted into their pharmaceutically usable salts.

5. Process for the preparation of compounds according to Claim 1, characterised in that compounds of the formula

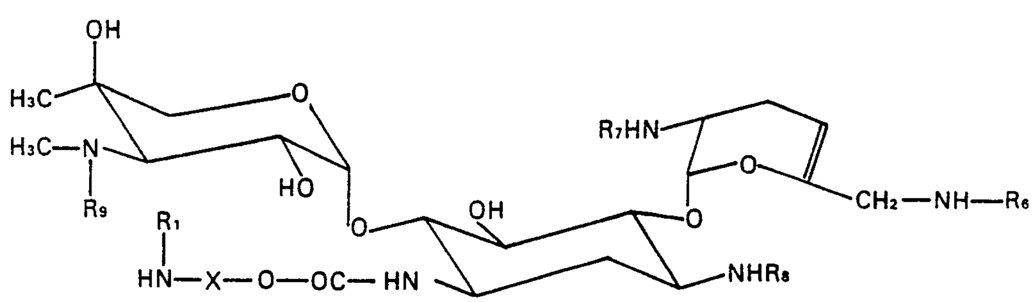

wherein

$R_1$, $R_6$, $R_7$, $R_8$, $R_9$ and X have the meaning indicated in Claim 4,

are reacted with carbonyl compounds of the formula

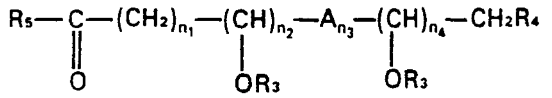

wherein

$R_3$, $R_4$, $R_5$, A, $n_1$, $n_2$, $n_3$ and $n_4$ have the meaning given in Claim 1,

in the presence of a hydrogen donor reducing agent and the protective groups $R_6$, $R_7$, $R_8$ and $R_9$ and, if appropriate, alkylidene groups which 2 radicals $R_3$ form together, are split off and the resulting products are optionally converted into their pharmaceutically usable salts.

6. Medicaments, characterised in that they contain a compound according to Claim 1 to 3

**Revendications**

1. Composés de formule:

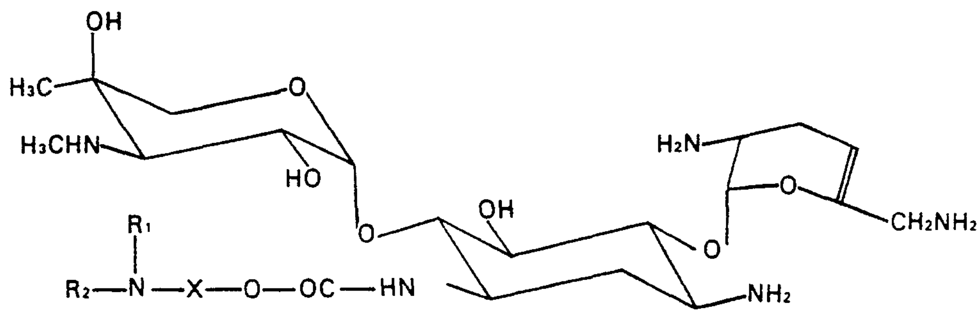

dans laquelle X représente un radical aliphatique à chaîne droite, à chaîne ramifiée ou cyclique, saturé ou insaturé et contenant 2 à 10 atomes de carbone,

$R_1$ représente un atome d'hydrogène, un groupe alkyle en $C_1$-$C_4$ ou un groupe benzyle, et

$R_2$ représente un radical de formule:

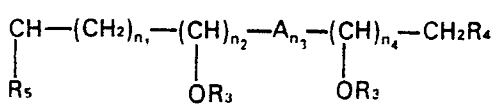

A représentant

$$—CH{=}CH—, \quad \left[— CH_{(2\text{-}n_5)} —(CH_2OR_3)_{n_5}\right]$$

$R_3$ représente un atome d'hydrogène, un groupe triarylméthyle, alkyle ou acyle, ou encore deux radicaux

$R_3$ ensemble représentent un groupe alkylidène,

$R_4$ représente un atome d'hydrogène ou un groupe $OR_3$,

$R_5$ représente un atome d'hydrogène ou un groupe alkyle en $C_1$-$C_4$ éventuellement substitué par 1-3 groupes hydroxy,

$n_1$ représente 0, 1, 2 ou 3,

$n_2$ représente 0, 1, 2, 3, 4 ou 5, et

$n_3$, $n_4$ et $n_5$ représentent chacun indépendamment l'un de l'autre 0, 1 ou 2,

la somme de $n_1$, $n_2$, $n_3$ et $n_4$ prenant des valeurs de 1 à 5, tandis que le nombre total de groupes $OR_3$ est de 1 à 6.

2. Composés suivant la revendication 1, dans lesquels X représente un groupe alkylène en $C_2$-$C_6$, tandis que $R_1$ représente un atome d'hydrogène.

3. Composés suivant la revendication 1, dans lesquels $R_3$ représente un atome d'hydrogène.

4. Procédé de préparation de composés suivant la revendication 1, dans lesquels $R_1$ représente un atome d'hydrogène, caractérisé en ce qu'on fait réagir, de façon connue en soi, un composé de formule:

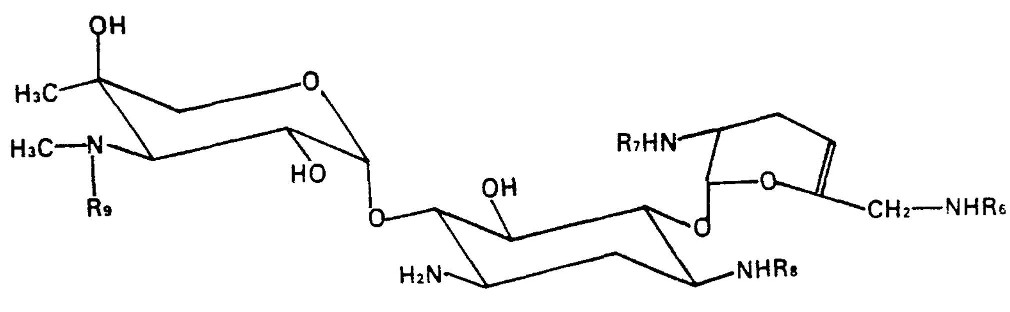

dans laquelle

$R_6$, $R_7$ et $R_8$ représentent -CO-$R_{10}$ ou -S-$R_{11}$, et

$R_9$ représente-S-$R_{11}$,

$R_{10}$ représentant un radical répondant à une des formules:

$— CHal_3$, $—(CH_2)_{n_6}B$, $—O—E$,

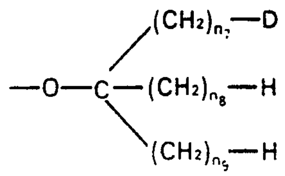

ou

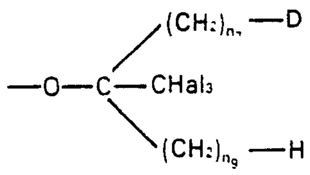

B et D représentent chacun un atome d'hydrogène ou un groupe phényle éventuellement substitué,
E représente un groupe phényle éventuellement substitué,
$n_6$ représente un nombre de 0 à 5,
$n_7$, $n_8$ et $n_9$ représentent un nombre de 0 à 5,
Hal représente un atome de fluor, de chlore ou de brome, et
$R_{11}$ représente un groupe triphénylméthyle, diphénylméthyle ou phényle éventuellement substitué,

avec un agent d'acylation de formule:

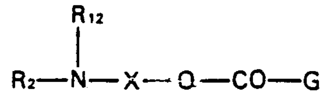

dans laquelle
$R_2$ et X ont les significations indiquées dans la revendication 1, et
G représente un groupe de départ, de préférence, un atome d'halogène tel qu'un atome de chlore et un atome de brome, un groupe azido, un groupe phénoxy éventuellement substitué ou un radical de formule:

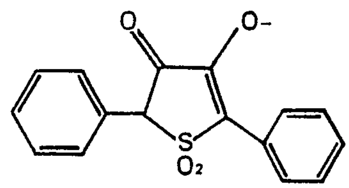

et $R_{12}$ représente $-CO-R_{10}$ ou $-S-R_{11}$,
on sépare, de la manière habituelle, les groupes protecteurs $R_6$, $R_7$, $R_8$, $R_9$ et $R_{12}$, ainsi qu'éventuellement des groupes alkylidène qui forment ensemble deux radicaux $R_3$ et on transforme éventuellement les composés obtenus en leurs sels pharmaceutiquement utiles.

5. Procédé de préparation de composés suivant la revendication 1, caractérisé en ce qu'on fait réagir des composés de formule:

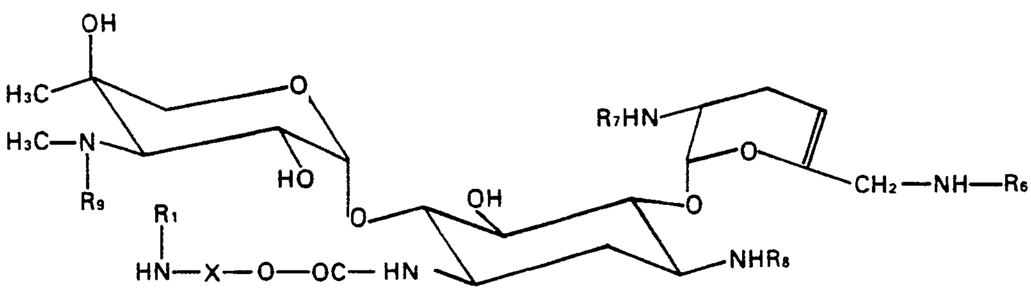

dans laquelle
$R_1$, $R_6$, $R_7$, $R_8$, $R_9$ et X ont les significations indiquées dans la revendication 4,
avec des composés carbonyle de formule:

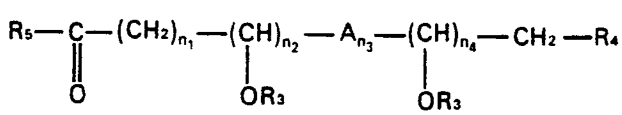

dans laquelle
$R_3$, $R_4$, $R_5$, A, $n_1$, $n_2$, $n_3$ et $n_4$ ont les significations mentionnées dans la revendication 1,
en présence d'un agent réducteur donneur d'hydrogène, puis on sépare les groupes protecteurs $R_6$, $R_7$, $R_8$ et $R_9$, ainsi qu'éventuellement des groupes alkylidène formant ensemble deux radicaux $R_3$ et on transforme éventuellement les produits obtenus en leurs sels pharmaceutiquement utiles.

6. Médicaments, caractérisés en ce qu'ils contiennent un composé suivant les revendications 1 à 3.